# EUROPÄISCHE PATENTSCHRIFT

(11) **EP 2 414 009 B1**
(45) Veröffentlichungstag und Bekanntmachung des Hinweises auf die Patenterteilung: **13.08.2014**
(21) Anmeldenummer: 10712429.9
(22) Anmeldetag: 31.03.2010
(51) Int. Cl.: A61M 5/315, A61M 5/24

(54) **ANORDNUNG ZUM APPLIZIEREN VON INSULIN AUS EINER PATRONE**
ARRANGEMENT FOR ADMINISTERING INSULIN FROM A CARTRIDGE
DISPOSITIF DESTINÉ À L'ADMINISTRATION D'INSULINE HORS D'UNE CARTOUCHE

(30) Priorität: 01.04.2009 DE 102009003721
(43) Veröffentlichungstag der Anmeldung: 08.02.2012
(60) Teilanmeldung: 14171237.2
(73) Patentinhaber: Emperra GmbH E-Health Technologies, 14469 Potsdam (DE)
(72) Erfinder: SCHILDT, Janko, 14467 Potsdam (DE); RÜHLE, Florian, 14482 Postdam (DE)
(74) Vertreter: Gulde & Partner
(86) Internationale Anmeldenummer: PCT/EP2010/054383
(87) Internationale Veröffentlichungsnummer: WO 2010/112575

(56) Entgegenhaltungen:
- EP-A1- 1 095 668
- WO-A1-93/10839
- WO-A1-97/17095
- WO-A1-98/42394

## Beschreibung

Die Erfindung betrifft eine Anordnung zum Applizieren einer ausgewählten Dosis Insulin oder anderer Medikamente aus einer Patrone durch Vorschub eines in der Patrone verschiebbar geführten Stopfens, enthaltend
(a) ein in Vorschubrichtung bewegliches, erstes Gewindelement zum Bewegen des Stopfens, und
(b) ein zum Einstellen der ausgewählten Dosis drehbar mit dem ersten Gewindeelement verschraubtes, zweites Gewindeelement, mit welchem der Hub des ersten Gewindeelements begrenzbar ist.

Solche Applikationsanordnungen werden insbesondere bei der Insulintherapie des Diabetes mellitus verwendet. Bei der Insulintherapie, insbesondere bei der intensivierten, konventionellen Insulintherapie und der konventionellen Insulintherapie, wird Insulin nicht in konstanten Mengen appliziert. Bei der konventionellen Insulintherapie wird zu bestimmten Tageszeiten Insulin appliziert. Der Tagesablauf des Patienten richtet sich nach diesen Zeiten. Bei der intensivietten, konventionellen Insulintherapie wird ein Grundbedarf an Insulin bereitgestellt, oft durch ein langsam und lang anhaltend wirkendes Insulin, das Basalinsulin. Zu den Malzeiten wird schnellwirkendes Insulin gespritzt. Die Dosis des schnellwirkenden Insulins richtet sich im Wesentlichen nach den aufgenommenen Kohlehydraten. Die Dosis wird also in Abhängigkeit von den äußeren Umstanden spezifisch ausgewählt. Solche Umstände sind beispielsweise die Tageszeit, der Bewegungsumfang, die Ernährung und dergleichen.

Diabetes mellitus kann schwerwiegende Langzeitfolgen und Körperschädigungen haben. Diese können durch eine angepasste Insulintherapie, vorzugsweise eine intensivierte, konventionelle Insulintherapie wesentlich gemindert werden. Eine falsche Dosierung kann aber auch kurzzeitige Folgen wie Hypoglykämie-Zustände haben. Eine möglichst gute Anpassung der Dosis an die jeweiligen Umstände ist daher besonders erstrebenswert.

Aus diesem Grund werden die Diabeles-mellitus-Patienten zur genauen Protokollführung über ihre Lebensgewohnheiten und verabreichte Dosen an Insulin angehalten. Diese Protokollführung ist aufwändig. Ein solches Protokoll umfasst üblicherweise den gemessenen Blutzuckerwert, die aufgenommene Menge an Kohlenhydraten, die gespritzte Insulindosis sowie das Datum und die Uhrzeit. Aus dem Protokoll kann der behandelnde Arzt oder der Patient die jeweilige Dosis ermitteln oder anpassen.

Die Verabreichung des Insulins erfolgt mittels einer Injektionshilfe (auch als Pen bezeichnet), die eine Injektionsnadel und eine Patrone für das Medikament oder eine Insulinpumpe mit Patrone aufweist. Die Insulinpumpe wird dauerhaft eingesetzt, während ein Pen bei einzelnen Applikationsvorgängen eingesetzt wird.

### Stand der Technik

Bekannte Pens ähneln äußerlich einem dickeren Kugelschreiber. Sie umfassen ein Gehäuse und eine Patrone. Unter einer Patrone wird hier eine zylindrische Ampulle verstanden, die auf einer Seite mit einer durchstechbaren Membran versehen ist. Die andere Seite ist mit einem verschiebbaren Stopfen verschlossen. In der Patrone ist das Medikament enthalten. Die Patrone ist gewöhnlich auswechselbar. Es sind aber auch Anordnungen bekannt, die als Einweg-Pens ausgebildet sind. Die Patronen und deren Inhalt, Abmessungen und Handhabung sind nicht normiert. Eine Patrone eines Herstellers kann daher im Regelfall nicht in den Pen eines anderen Herstellens eingesetzt werden.

Ein Pen umfasst eine Dosiereinrichtung. An einem Dosierknopf wird die erforderliche Dosis eingestellt. Diese wird dann mittels einer Injektionsnadel ins subkutane Fettgewebe gespritzt. Bei einigen Pens wird die eingestellte Dosis statt mit einer mechanischen Anzeige auf dem Dosierknopf mit elektrischer Energie auf einem Display angezeigt. Das Display wird mit einer Knopfzelle betrieben. Der Patient kann die Dosis einstellen und in seinem Diabetikertagebuch notieren. Das Diabetikertagebuch wird in regelmäßigen Abständen einem Arzt vorgelegt. Zusammen mit anderen Körperwerten, z. B. Blutzuckerwerten, Körpermaßen und Blutdruck kann der Arzt die weitere Therapie ausarbeiten.

Die Protokollführung ist bei den bekannten Methoden aufwändig. Es kann zu Nachlässigkeiten des Patienten kommen. Es besteht die Gefahr der bewussten oder unbewussten Angabe falscher Insulindosen. Dies kann beispielsweise vorkommen, wenn sich der Patient entgegen den ärztlichen Empfehlungen verhält. Eine fehlerhafte Selbstbehandlung möchte der Patient üblicherweise nicht offenlegen. Dann stimmen die Angaben auf dem Protokoll nicht und die Anpassung der zukünftigen Therapie ist falsch. Der Patient muss mit gravierenden Akut- oder Langzeitfolgen rechnen.

DE 101 47 973 A1 offenbart eine Anordnung, bei welcher Sensoren zum Erkennen einer Patrone vorgesehen sind, um Verwechselungen zu verhindern. Dazu sind in der Anordnung Sensoren angebracht, die mit Erkennungselementen an einer Ampulle zusammenwirken. Dadurch lässt sich das Medikament in einer Ampulle eindeutig auslesen.

In DE 10 2005 018 305 A1 ist eine Applikationsanordnung offenbart, bei der ein Dosierknopf gegen unbeabsichtigtes Betätigen geschützt ist. Ein Einstellring an einem Ende der Applikationsanordnung ist zum Einstellen einer Dosis vorgesehen. Der Dosierknopf ist separat von einem Einstellring angeordnet und dient nur dem Applizieren einer eingestellten Dosis. Am Einstellring kann die zu applizierende Dosis eingestellt werden. Das Betätigungselement ist gleitend im Einstellring gelagert. Durch Drehen des Einstellrings verschiebt sich der Dosierknopf in axialer Richtung. Das Betätigungselement ragt nie über den Einstellring hinaus. Dies dient als Sicherhcitsmechanismus um zu verhindern, dass der Dosiervorgang versehentlich ausgelöst wird.

Es ist nachteilig beim Stand der Technik, dass der Patient für Insuline verschiedener Hersteller unterschiedliche Pens verwenden muss. Dies zwingt den Patienten zum erlernen einer neuer Handhabung. Es ist weiterhin nachteilig, dass verschiedene Insulin-Pens unterschiedliche Sicherheitsmechanismen aufweisen, die ein versehentliches Bedienen verhindern.

EP 1 095 668 A1 offenbart einen Pen, welcher für die Verwendung mit unterschiedlichen Patronen vorgesehen ist. Ein Dosiersensor ermittelt aus einer an der Patrone vorgesehenen Information beispielsweise einem Farbcode zusammen mit einem Mikroprozessor die erforderliche Anzahl an Umdrehungen, welche den Vorschub eines Gewindeelements und somit den Hub des Stopfens in der Patrone bestimmen.

### Offenbarung der Erfindung

Es ist Aufgabe der Erfindung, eine Anordnung der eingangs erwähnten Art zu schaffen, die wirtschaftlicher und leichter handhabbar ist. Es ist weiterhin Aufgabe der Erfindung, eine Anordnung zu schaffen, deren Umgang sicherer ist.

Erfindungsgemäß wird die Aufgabe bei einer Applikationsanordnung der eingangs genannten Art dadurch gelöst dass
(a) das erste und das zweite Gewindeelement in einer Adapteranordnung zur Anpassung von Patronen unterschiedlicher Abmessungen und Inhalte enthalten ist, wobei
(b) die Gewindesteigung des ersten und zweiten Gewindeelements an die Abmessungen und/oder den Inhalt der Patrone angepasst ist.

Insuline verschiedener Hersteller werden in unterschiedlichen Patronengrößen angeboten. Durch die Verwendung einer erfindungsgemäßen Anordnung kann der Benutzer den gleichen Pen für alle Insuline verschiedener Hersteller verwenden. Dies reduziert die Anschaffungskosten, weil bei einer Änderung der Medikation kein neuer Pen angeschafft werden muss. Der Benutzer hat den Vorteil, dass er sich nicht an die Handhabung einer neuen Applikationsanordnung gewöhnen muss. Die Handhabung wird dadurch erleichtert und sicherer.

Patronen unterschiedlicher Anbieter variieren in den äußeren Abmessungen. Daher kann die Patrone eines anderen Anbieters für gewöhnlich nicht in andere Pens nach dem Stand der Technik eingesetzt werden. Eine Adapteranordnung gleicht dies aus. Dann können Patronen unterschiedlicher Hersteller verwendet werden. Die Steigung der Gewindeelemente bestimmt den Vorschub des Stopfens. Dadurch wird sichergestellt, dass die applizierte Dosis nicht von der Form der verwendeten Patrone abhängt. Jeder Patronenart ist eine eigene Gewindesteigung zugeordnet.

Die Erfindung beruht auf der Erkenntnis, dass die Integration von Gewindeelementen mit angepasster Gewindesteigung die Verwendung unterschiedlicher Patronen ermöglicht. Die Patronen können unterschiedliche Abmessungen und Inhalte haben. Entsprechend ist der Vorschub des Stopfens bei unterschiedlichen Patronen unterschiedlich.

Vorzugsweise umfasst die Anordnung einen drehbar gelagerten Dosierknopf und Mittel zum Übertragen einer Rotationsbewegung des Dosierknopfes auf das zweite Gewindeelement. Der Dosierknopf kann insbesondere arretierbar sein. In einer besonders bevorzugten Ausgestaltung der Erfindung ist ein Gehäuse vorgesehen, in welchem der Dosierknopf gegen die Federkraft einer Druckfeder in dem Gehäuse versenk- und dort lösbar einrastbar ist. Der Dosierknopf arbeitet dann ähnlich wie eine Kugelschreibermechanik. Zur Benutzung wird der Dosierknopf gelöst und von der Feder nach außen gedrückt. Dann kann er in gewünschter Weise gedreht werden. Mit der Drehung wird die Dosis eingestellt. Die Drehung wird dabei auf das Gewindeelement übertragen. Zum Applizieren wird der Dosierknopf dann gedrückt. Wenn die Anordnung nicht benutzt wird, ist der Dosierknopf versenkt und gegen unbeabsichtigtes Betätigen gesichert.

Bei einer Ausgestaltung der Erfindung ist vorgesehen, dass
(a) das erste Gewindeelement von einer Gewindestange mit Außengewinde gebildet ist,
(b) die Adapteranordnung eine Hülle umfasst, die einseitig zur Aufnahme einer Patrone offen ist und am anderen Ende einen Durchbruch aufweist, durch den die Gewindestange verdrehsicher geführt ist, und
(c) das zweite Gewindeelement von einer außerhalb der Hülle auf die Gewindestange aufgeschraubten Mutter gebildet ist, deren axiale Lage durch Verdrehen einstellbar ist.

Eine solche Anordnung ist kostengünstig herstellbar und einfach aufgebaut. Es ist aber selbstverständlich auch denkbar, eine Hohlwelle mit einem Gewinde oder ähnliches zu verwenden. Statt einer Hülle kann auch jedes andere Halteelement für einen Adapter eingesetzt werden.

Vorteilhafterweise sind Sicherungsmittel zum Blockieren einer Bewegung der Gewindestange entgegen der Vorschubrichtung vorgesehen.

Die Sicherungsmittel dienen dazu, dass sich der Adapter nur einmal verwenden lässt. Durch die Sicherungsmittel kann das Gewindeelement nicht mehr in die Ausgangsposition zurückgeführt werden. Wenn einmal Insulin einer Patrone appliziert wurde, kann keine neue Patrone eingesetzt werden. Der Adapter muss ausgewechselt werden. Dadurch, dass der Adapter nur einmal verwendet werden kann, ist der mechanische Verschleiß an den Komponenten minimal. Die Applikationsanordnung arbeitet genauer und zuverlässiger.

Vorteilhafterweise sind die Sicherungsmittel von einer Sicherungsscheibe gebildet, die innerhalb der Hülle auf die Gewindestange aufgesteckt und mit der Patrone arretierbar ist.

Die Sicherungsmittel können von einer Scheibe gebildet sein, die mit einer Öffnung zwischen der Stirnseite der Patrone und der inneren Stirnseite der Hülle auf die Gewindestange aufgesteckt ist und welche im Bereich der Öffnung flexible Zungen aufweist, welche in das Gewinde der Gewindestange eingreifen.

Eine solche Sicherungsscheibe ist günstig in der Herstellung und zuverlässig in der Handhabung. Es versteht sich, dass die Sicherungsmittel auch an der Hülle vorgesehen sein können.

In einer besonders bevorzugten Ausgestaltung der Erfindung ist eine Sensoranordnung zum kontaktlosen Erfassen der eingestellten Dosis vorgesehen. Der Verzicht auf mechanische Teile verringert den Verschleiß und erhöht damit die Zuverlässigkeit der Applikationsanordnung. Dabei kann die Sensoranordnung enthalten:
(a) Magnete an einer der zum Einstellen der Dosis rotierenden Komponenten,
(b) Magnetsensoren zum Detektieren einer Rotationsbewegung der Magnete und
(c) Auswerte- und/oder Anzeigemittel zum Auswerten und/oder Anzeigen einer der detektierten Bewegung entsprechenden Dosis, die aus der detektierten Rotationsbewegung ermittelt wurde.

Magnete eignen sich besonders zum kontaktlosen Übertragen von Signalen, da Magnetfelder räumlich ausgedehnt sind. Sensoren können Magnetfelder detektieren ohne in mechanischem Kontakt zu den Magneten zu stehen. Die Magnetfelder von Magneten bleiben über einen sehr langen Zeitraum konstant, wodurch die Zuverlässigkeit der Applikationsanordnung erhöht wird.

Vorteilhafterweise sind Mittel zur Datums- und/oder Zeiterfassung und Mittel zur Erfassung der eingestellten Dosis vorgesehen. Durch das Abspeichern des Datums und/oder der Uhrzeit zusammen mit der eingestellten Dosis kann der Patient leicht nachvollziehen, wann er das letzte Mal Insulin appliziert hat. Bei Basalinsulinen ist es wichtig, genaue Zeiten einzuhalten zu denen Insulin appliziert wird. Dies ist durch die Angabe der verstrichenen Zeit seit der zuletzt applizierten Dosis realisiert. Mit einer solchen Einrichtung kann der Patient kontrollieren, wann er die letzte Dosis appliziert hat. Das ist hilfreich, wenn der Patient den Verlauf des Blutzuckerwertes seit der letzten Insulindosis überprüfen möchte. Weiterhin kann auch die eingestellte Insulindosis gespeichert werden. Dann kann der Patient später nachvollziehen, ob die applizierte Dosis richtig berechnet war.

Mittels eines Senders können Datum und/oder Zeit zusammen mit dem Signal der applizierten Medikamentenmenge zu einem Empfänger übertragen werden. Die Daten können auch unabhängig voneinander erfasst und übertragen werden.

Zur Verringerung der Übertragungswege kann das Signal, z. B. über einen Kurzstreckenfunk-Standard (z. B. Bluetooth^{®}) an ein Mobilfunktelefon übertragbar sein. Die weitere Übertragung kann dann mittels der üblichen Mobilfunknetze erfolgen. Es ist selbstverständlich auch jede andere Übertragung denkbar. So kann die Übertragung auch auf einen Empfänger erfolgen, der mit dem Internet verbunden ist. Auch GSM und dergleichen sind zur Übertragung des Signals geeignet. Die übertragenen Daten können verschlüsselt sein.

Die Anordnung kann auch einen Empfänger zum Empfangen von Daten aufweisen. Über diesen können Empfangsbestätigungen, Warnungen oder sonstige Daten empfangen werden. Insbesondere erlaubt eine Anordnung mit einem Empfänger eine Kommunikation mit dem behandelnden Arzt auch dann, wenn eine anderweitige Kommunikation nicht möglich ist.

Die Anordnung kann ein Display zur Anzeige von Warnungen, Übertragungsdaten, Statusinformationen und dergleichen aufweisen. Dies erleichtert die Handhabung.

Die Anordnung kann weiterhin Mittel zur akustischen Ein- und/oder Ausgabe von Daten umfassen. Im Krankheitsverlauf können bei Diabetikern Sehstörungen auftreten. Die Verwendung einer akustischen Ein- und/oder Ausgabe von Daten gewährleistet, dass auch diese Patienten eine Injektionshilfe bedienen können.

An der Anordnung kann ein Tastfeld zur Ein- und Ausgabe von Daten und Dosiseinstellungen vorgesehen sein. Dieses Tastfeld kann für Blinde ausgelegt sein, so dass auch Blinde in die Lage versetzt werden die Applikationsvorrichtung zu nutzen.

Der Energiespeicher kann auswechselbar ausgestaltet sein, so dass jederzeit die Funktionsfähigkeit gewährleistet ist. Alternativ kann die Anordnung einen schnurlos aufladbaren und/oder auswechselbaren Energiespeicher aufweisen. Dann kann sie ohne weiteres ständig beim Patienten verbleiben. Der Energiespeicher kann ähnlich wie eine elektrische Zahnbürste über Induktion schnurlos aufgeladen werden.

Die Anordnung kann in einem Netzwerk zur Übertragung, Speicherung und Verarbeitung von Daten bezüglich der Dosierung von Medikamenten verwendet werden. Das Netzwerk kann eine Applikationsanordnung mit jeweils einem Sender zum Senden von Daten bezüglich der eingestellten Menge eines Medikaments, einen Empfänger zum Empfangen des Signals und eine Datenverarbeitungsanlage zum Speichern und Verarbeiten der empfangenen Daten aufweisen.

In der Datenverarbeitungsanlage können personenspezifische Daten gespeichert und Mittel zum Verarbeiten der von dem Sender übertragenen Daten zusammen mit diesen personenspezifischen Daten vorgesehen sein.

Das Netzwerk kann eine Basisstation umfassen, welche die Daten der Applikationsanordnung erfasst und an die Datenverarbeitungsanlage überträgt. Die Übertragung der Signale der Applikationsanordnung an die Basisstation kann drahtlos erfolgen. Die Übertragung von der Basisstation zur Datenverarbeitungsanlage kann über ein Mobilfunknetz erfolgen.

Ausgestaltungen der Erfindung sind Gegenstand der Unteransprüche. Ausführungsbeispiele sind nachstehend unter Bezugnahme auf die beigefügten Zeichnungen näher erläutert.

### Kurze Beschreibung der Zeichnungen

- Fig.1: ist ein Längsschnitt durch eine Applikationsanordnung für Insulin mit mechanischer Einstellung der Dosis.
- Fig.2: zeigt einen Adapter mit einer Patrone zum Anpassen an die Applikationsanordnung für Insulin aus Figur 1 im Detail.
- Fig.3: ist ein Querschnitt durch die Applikationsanordnung aus Figur 1 quer zu deren Längsachse.
- Fig.4: zeigt schematisch den Aufbau eines Netzwerkes für Applikationsanordnungen entsprechend Figur 1 mit einer Datenverarbeitungsanlage, auf der Patientendaten aufbewahrt werden.
- Fig.5: zeigt Querschnitte durch drei Ebenen der Anordnung aus Figur 1 in unterschiedlichen Stellungen der Dosiermittel.
- Fig.6: ist eine Explosionsdarstellung der Mechanik der Applikationsanordnung aus Figur 1, welche die Bewegung des Dosierknopfes entlang der Längsachse der Anordnung steuert.
- Fig.7: ist ein Längsschnitt durch ein alternatives Ausführungsbeispiel einer Applikationsanordnung, bei welcher die Dosis kontaktlos erfasst wird.
- Fig.8: zeigt einen Adapter mit einer Patrone zum Anpassen an die Applikationsanordnung für Insulin aus Figur 7 im Detail.
- Fig. 9: illustriert den Rastmechanismus bei der Dosierung von Insulin mit einer Anordnung aus Figur 7.
- Fig. 10 a: ist eine Explosionsdarstellung des patronenseitigen Teils des Ausführungsbeispiels aus Figur 7.

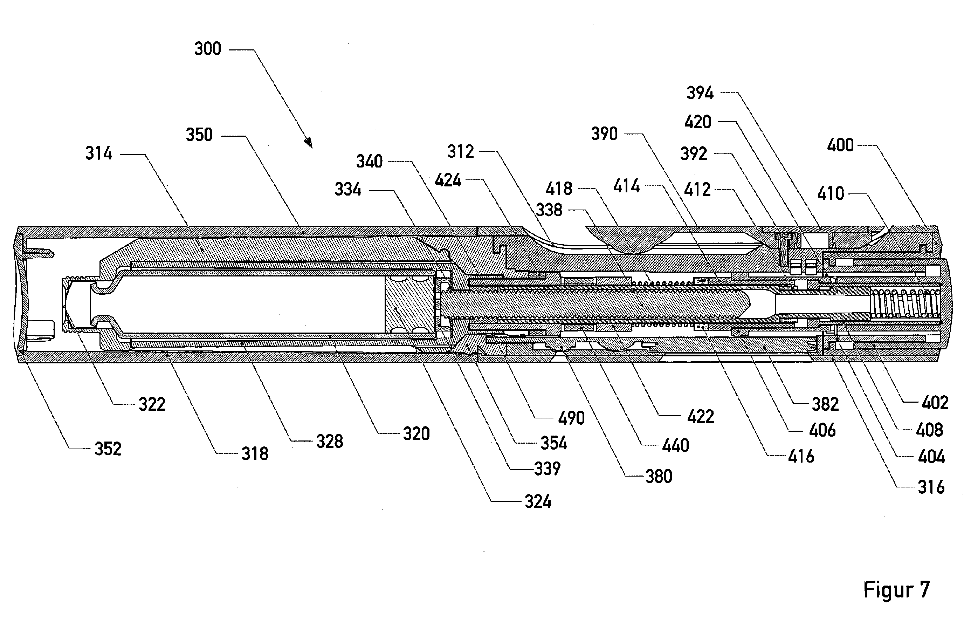

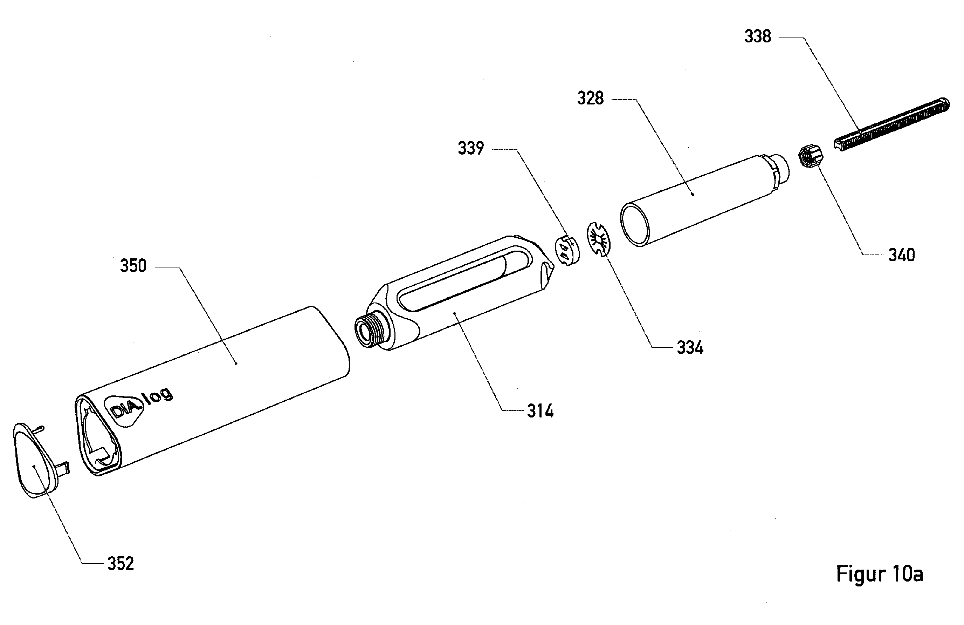

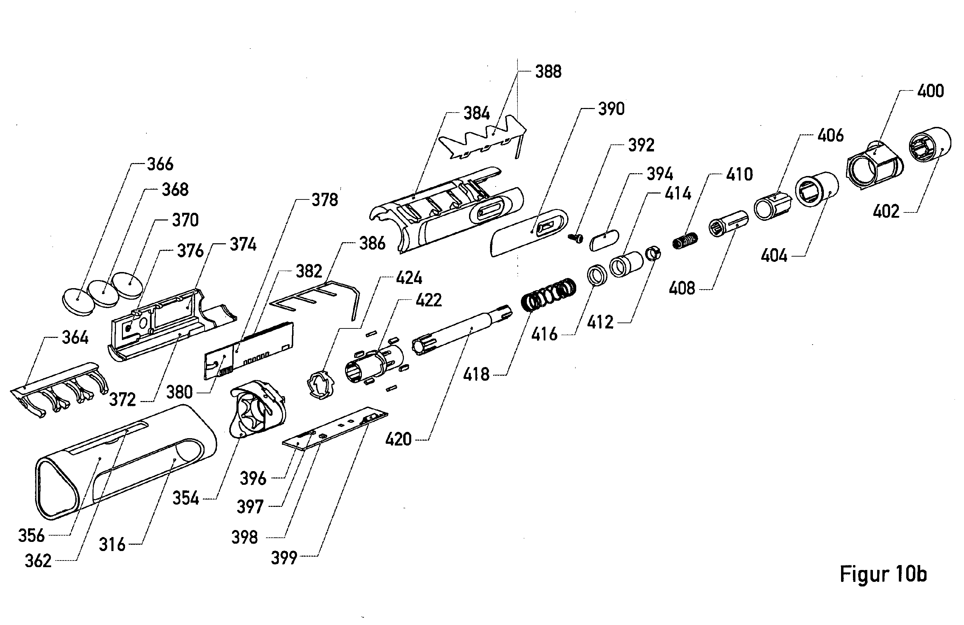

- Fig. 10 b: ist eine Explosionsdarstellung des Dosierknopf-seitigen Teils des Ausführungsbeispiels aus Figur 7.

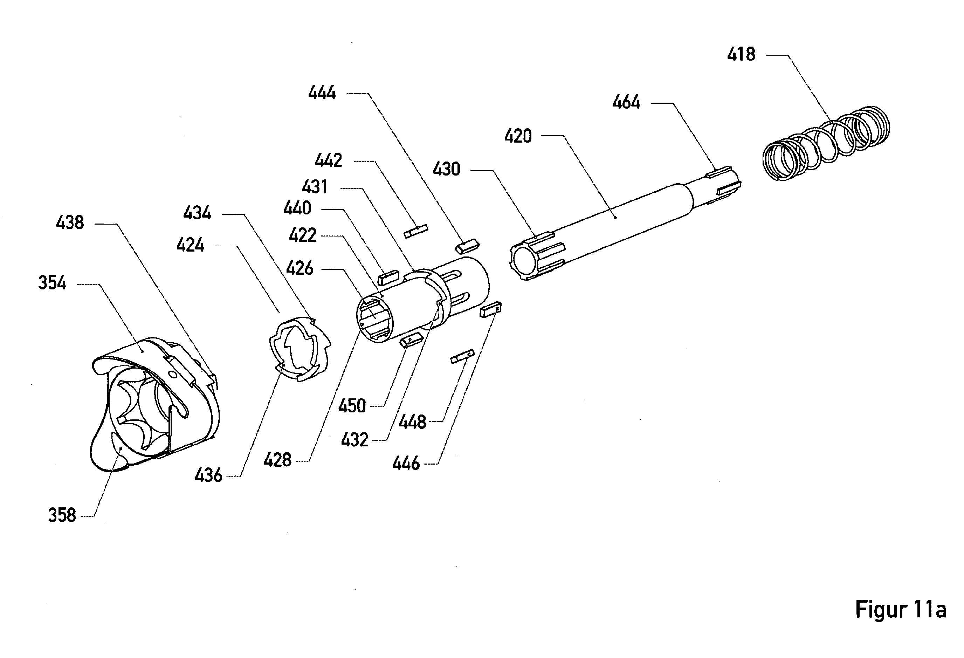

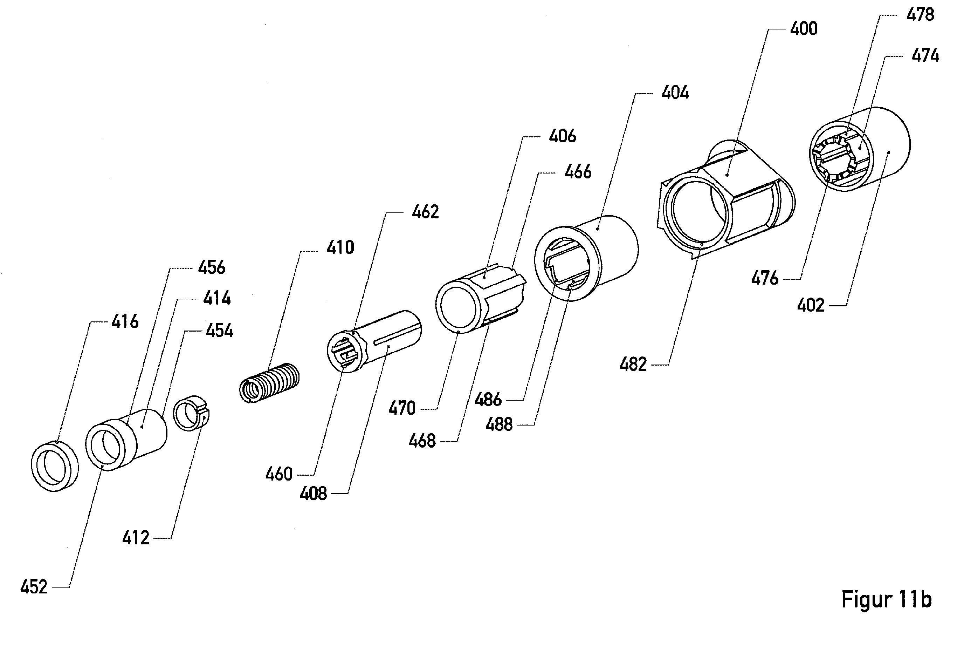

- Fig.11 a: ist eine Explosionsdarstellung eines Teils der Mechanik aus Figur 10b im Detail.
- Fig.11 b: ist eine Explosionsdarstellung eines weiteren Teils der Mechanik und des Dosierknopfes aus Figur 10b.

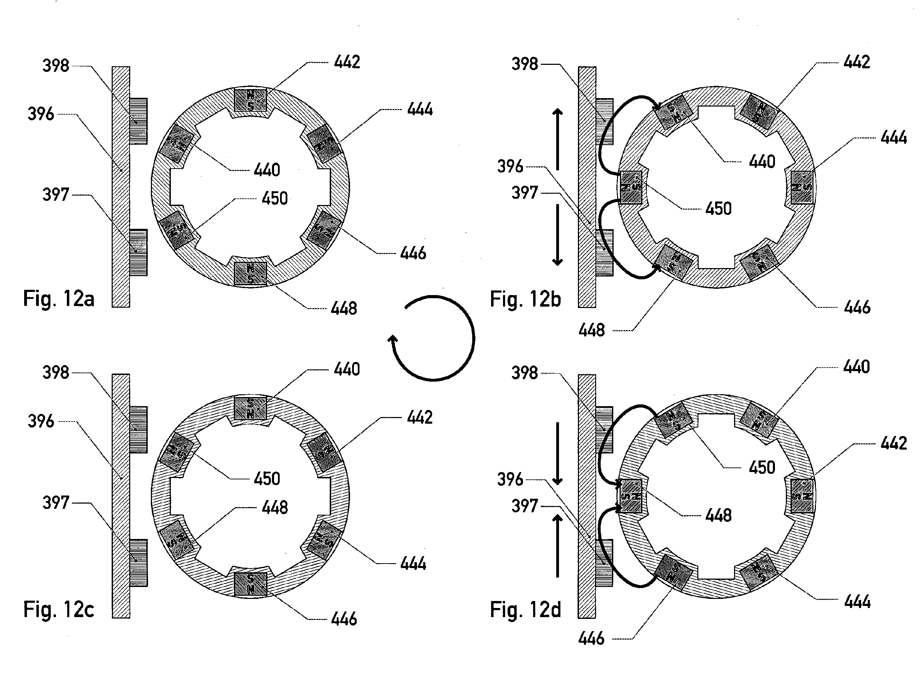

- Fig.12 a-d: sind Querschnitte quer zur Längsachse einer Applikationsanordnung aus Figur 7 in verschiedenen Stellungen.

### Beschreibung der Ausführungsbeispiele

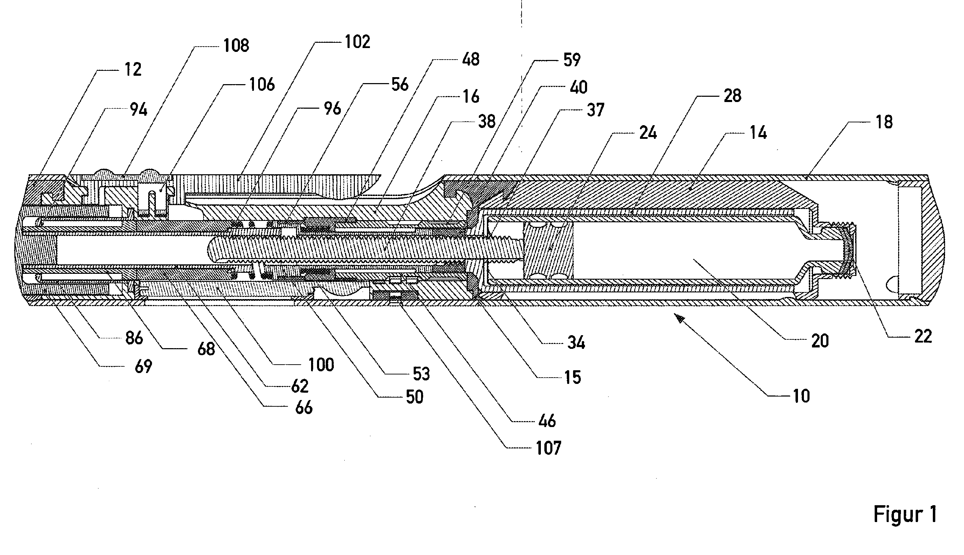

In Figur 1 ist mit 10 allgemein eine Applikationsanordnung für Insulin bezeichnet. Die Applikationsanordnung 10 weist ein langgestrecktes Gehäuse aus Kunststoff auf. Das Gehäuse besteht aus einem vorderen Gehäuseteil 14, einen hinteren Gehäuseteil 16, einem zwischen dem vorderen und dem hinteren Gehäuseteil 14 bzw. 16 angeordneten Zwischenstück 15 und einem Abschlussteil 12. Der vordere Gehäuseteil 14 wird von einer langgestreckten Abdeckkappe 18 abgedeckt.

Im vorderen Gehäuseteil 14 ist eine im wesentlichen zylindrische Patrone 20 angeordnet. Die Patrone 20 ist mit Insulin gefüllt. Die Patrone 20 weist eine Membran 22 auf. In die Membran 22 kann zum Applizieren des Insulins eine Nadel (nicht dargestellt) eingeführt werden.

Die Patrone 20 weist außerdem einen in axialer Richtung beweglich geführten Stopfen 24 auf. Bei Patronen kann der Durchmesser je nach Hersteller variieren. Zum Ausgleich wird eine nachstehend im Detail beschriebene Adapteranordnung mit einer austauschbaren Hülle 28, einer Gewindestange 38, einer Mutter 40 und einer Scheibe 34 eingesetzt. Für unterschiedliche Patronen werden zugehörige, unterschiedliche Adapteranordnungen eingesetzt. Die Patrone 20 sitzt rutschfest in der Adapteranordnung. Am hinteren Gehäuseteil 16 befindet sich ein noch zu beschreibender Dosierknopf 69.

Die Adapteranordnung umfasst eine Hülle 28. Die Patrone 20 ist in der Hülle 28 angeordnet. Die Hülle 28 ist im Wesentlichen als Hohlzylinder ausgebildet. Der Innendurchmesser der Hülle 28 ist dem Außendurchmesser der Patrone 20 angepasst. Der Außendurchmesser der Hülle 28 entspricht dem Durchmesser eines zylindrischen Hohlraums im Inneren des vorderen Gehäuseteils 14. Die Hülle 28 dient als Ausgleich für verschiedene Patronenformen.

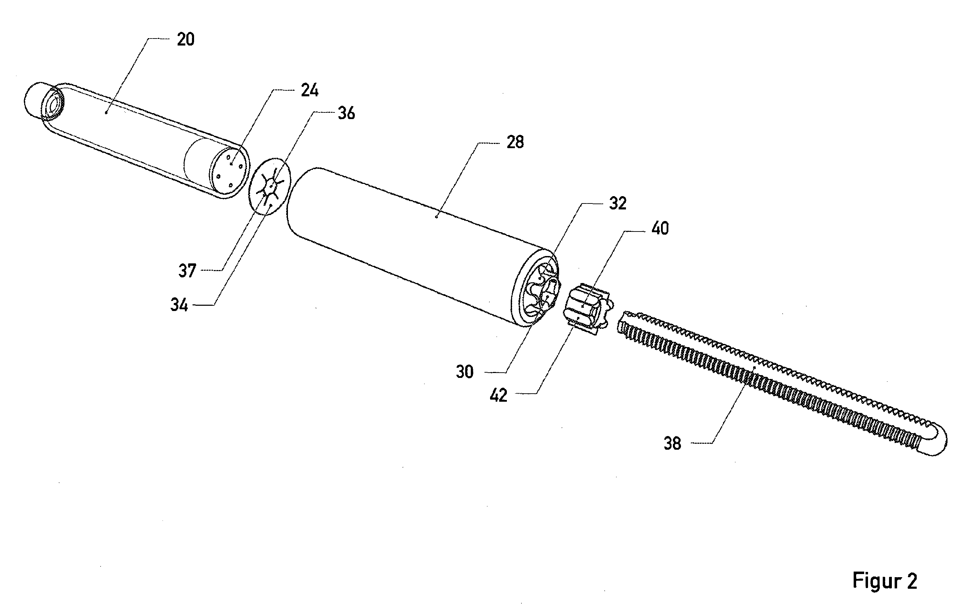

Die Komponenten 28, 38, 34, 40 der Adapteranordnung sind zusammen mit einer Patrone 20 in Figur 2 im Detail dargestellt. Die Hülle 28 ist an einem der Abdeckkappe 18 zugewandten Ende offen, so dass die Patrone 20 bei geöffnetem Gehäuse 14 eingeführt werden kann. Das dem Dosierknopf 69 zugewandte Ende der Hülle 28 ist geschlossen und weist einen mittigen Durchbruch 30 auf. Der Durchbruch 30 ist eine kreisrunde Öffnung mit drei nach innen ragenden Radialvorsprüngen. Die Hülle 28 weist auf der dosierknopfseitigen Stirnseite außen axiale Erhebungen 32 auf, die formschlüssig in Vertiefungen im Zwischenstück 15 eingreifen. Dadurch wird die Hülle 28 gegen Verdrehen gesichert.

Die Scheibe 34 besteht aus Blech und ist innen vor dem dosierknopfseitigen, geschlossenen Ende der Hülle 28 angeordnet. Die Scheibe 34 wird zwischen der Patrone 20 und dem geschlossenen Ende der Hülle 28 eingeklemmt. Dies ist in Figur 1 dargestellt. In Figur 2 ist die Scheibe zur besseren Veranschaulichung separat außerhalb der Hülle 28 dargestellt. Die Scheibe 34 weist ebenfalls eine mittige Öffnung 36 auf. Die Öffnung 36 ist sternförmig ausgebildet. Dadurch entstehen kleine, konzentrisch angeordnete, federnde Blechzungen 37. Die Anzahl der federnden Blechzungen 37 ist abhängig von der Symmetrie der sternförmigen Öffnung 36. Im vorliegenden Fall weist die Scheibe 34 sechs Blechzungen 37 auf. Die Gewindestange 38 ist von oben in Figur 1 durch den Durchbruch 30 und die Öffnung 36 in der Scheibe 34 axialbeweglich geführt.

Die Gewindestange 38 weist einen äußeren Umriss auf, der der Form des Durchbruchs 30 entspricht. Damit ist die Gewindestange 38 gegen Verdrehen gegenüber dem Gehäuse gesichert.

Die Gewindestange 38 weist ein Außengewinde auf. Die federnden Blechzungen 37 greifen in die Gewindegänge der Gewindestange 38. Bei einer Axialbewegung der Gewindestange 38 in Richtung der Patrone 20 werden die Blechzungen 37 leicht verbogen. Eine Bewegung der Gewindestange 38 in umgekehrter Richtung wird durch die Blechzungen 37 verhindert. Die Gewindestange 38 kann sich nur noch axial in Richtung der Patrone 20 bewegen. Die Gewindestange 38 drückt auf den Stopfen 24 der Patrone 20. Bei einer Axialbewegung der Gewindestange 38 und damit des Stopfens 24 nach unten in Figur 1 wird das Insulin wie bei einer Spritze durch die Injektionsnadel gepresst.

Auf der Gewindestange 38 ist außerhalb der Hülle 28 eine Mutter 40 mit Innengewinde aufgeschraubt. Die Mutter 40 weist auf der Außenseite radial angeordnete Mitnehmer 42 auf, die auch als Führung dienen. Vor dem Einsatz bei voller Patrone liegt die Mutter 40 am dosierknopfseitigen Ende der Hülle 28 an. Dies ist in Figur 1 dargestellt. Durch drehen der Mutter 40 bewegt sich die Mutter 40 relativ zum Gehäuse nach oben in Figur 1. Dabei entsteht ein Spalt zwischen der Mutter 40 und der gehäusefesten Hülle 28. Der Stopfen 24 wird dabei noch nicht beeinflusst. Zum Applizieren von Insulin wird Druck auf die Gewindestange 38 und damit auf den Stopfen 24 ausgeübt. Die Gewindestange 38 wird dabei in Richtung der Abdeckkappe 18 nach unten in Figur 1 geschoben.

Das in Figur 1 obere Ende der Hülle 28 bildet einen Anschlag für die Bewegung der Gewindestange 38 mit der Mutter 40. Der beim Drehen der Mutter 40 erzeugte Spalt entspricht dem Hub des Stopfens und ist ein Maß für die applizierte Insulindosis.

Die Steigung des Gewindes der Gewindestange 38 ist der Geometrie der Patrone 20 und dem verwendeten Insulin angepasst. Eine Patrone mit geringem Durchmesser erfordert bei gleichem Insulin und gleichem Volumen eine größere Steigung als eine Patrone größeren Durchmessers. Eine Drehung auf der Gewindestange 38 entspricht einem bestimmten Hub und somit der applizierten Insulindosis.

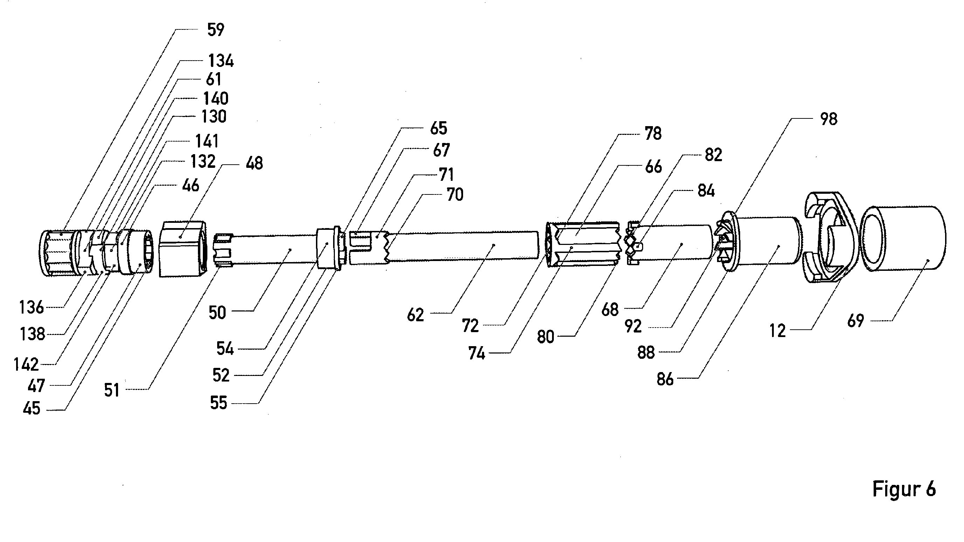

Die Dosierung über Drehung der Mutter 40 erfolgt mittels einer nachfolgend beschriebenen Dosiereinrichtung. Die Dosiereinrichtung ist im hinteren Gehäuseteil 16 angeordnet. Dies ist in Figur 1 und 6 zu erkennen. Die Adapteranordnung und die Dosiereinrichtung dienen zum Einstellen der Dosis.

Die Dosiereinrichtung umfasst eine Hülse 46 mit einem zylindrischen Ansatz 47. Die Hülse 46 ist mit dem zylindrischen Ansatz 47 in einer Lagerbuchse 48 drehbar gelagert. Die Innenwand der Hülse 46 weist axiale Nuten 45 auf. Die Mitnehmer 42 der Mutter 40 greifen in die Nuten 45 und sind dort axialbeweglich geführt.

Eine Drehbewegung der Hülse 46 wird so auf die Mutter 40 übertragen. Die Mutter 40 bewegt sich auf der unverdrehbaren Gewindestange 38 in axialer Richtung. Koaxial sitzt eine Hohlwelle 50 axialbeweglich im Inneren der Hülse 46. Die Hohlwelle 50 weist an seinem in der Hülse 46 versenkten Ende radial nach außen vorstehende, sich in axialer Richtung entlang des Umfangs erstreckende Vorsprünge 51 aus, die ebenfalls in die Nuten 45 in der Innenwand der Hülse 46 eingreifen. Die Hülse 46 folgt somit einer Drehbewegung der Hohlwelle 50.

Die Hohlwelle 50 weist an der dem Dosierknopf 69 zugewandten Seite eine Verdickung 52 entlang des Umfangs auf. Die Verdickung 52 erstreckt sich über einen zylindrischen Bereich 54, der von einem umlaufenden Rand 55 mit größerem Durchmesser abgeschlossen wird. Die Verdickung 52 dient als Widerlager für eine koaxial um die Hohlwelle 50 angeordnete Druckfeder 53. Die Druckfeder 53 ist in Figur 1 dargestellt. Das patronenseitige Widerlager der Druckfeder 53 wird von der Lagerbuchse 48 gebildet.

Die Druckfeder 53 drückt die Hohlwelle 50 aus der Hülse 46 heraus. Der Weg wird durch eine Auslösefeder 96 begrenzt, gegen die der umlaufende Rand dosierknopfseitig anschlägt. Die Auslösefeder ist in Figur 1 gezeigt. Zwischen der Mutter 40 und der Hohlwelle 50 ist ein Zwischenraum.

Wenn der Patient den Stab 50 dreht, verschiebt sich die Mutter 40 in der Hülse 46 in Richtung des Dosierknopfes 69. Dadurch reduziert sich der Zwischenraum zwischen Hohlwelle 50 und Mutter 40. Gleichzeitig vergrößert sich der Spalt zwischen dem in Figur 1 oberen Ende der Hülle 28 und der Mutter 40. Durch Ausüben einer Kraft auf die Hohlwelle 50 nach unten in Figur 1 wird die Druckfeder 53 zusammengedrückt. Der Zwischenraum zwischen Hohlwelle 50 und Mutter 40 verringert sich. Sobald die Hohlwelle 50 die Mutter 40 berührt, bewegt sich die Mutter 40 bei fortgesetzter Krafteinwirkung auf die Hohlwelle 50 in Richtung der Abdeckkappe 18. Da die Mutter 40 über das Gewinde mit der Gewindestange 38 verbunden ist, wird die Gewindestange 38 axial in Richtung der Abdeckkappe 18 verschoben bis die Mutter 40 am dosierknopfseitigen Ende der Hülle 28 anschlägt.

Der Bereich 54 ist von einem leitfähigen Rohr 56 umgeben. Das leitfähige Rohr 56 ist in Figur 1 dargestellt. Auf der dem Dosierknopf 69 zugewandten Seite der Lagerbuchse 48 sind Metallkontakte angebracht. Durch Zusammenwirken mit dem leitfähigen Rohr 56 wird ein Schalter gebildet. Das Schließen des Schalters signalisiert, dass eine Insulindosis appliziert wurde. Das Öffnen des Schalters wird als Einschaltsignal verwendet. In einem nicht gezeigten Ausführungsbeispiel ist ein magnet-induzierter Sensormechanismus vorgesehen, welcher durch Bewegung eines auf der Lagerbuches 48 befestigten Magneten an einem auf einer Leierplatte befestigten Magnetsensors vorbei diese genannten Schaltvorgänge realisiert.

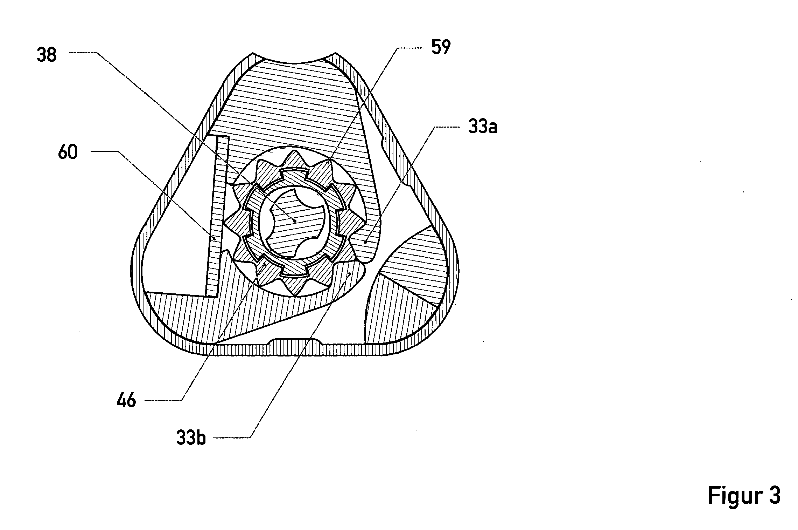

Die Hülse 46 ist auf der Außenseite in drei Bereiche unterteilt. Ein erster Bereich weist radial umlaufend mehrere Vorsprünge 59 auf. Am hinteren Gehäuseteil 16 vorgesehene, bewegliche Rastelemente 33 a und 33 b (in Figur 3 dargestellt) rasten in die Vorsprünge 59 ein. Dadurch wird die Drehbewegung der Hülse 46 in Winkelschritte unterteilt. Die Einteilung ist so gewählt, dass jedes Einrasten in die nächste Position einer Drehung der Hohlwelle 50 um 30° entspricht.

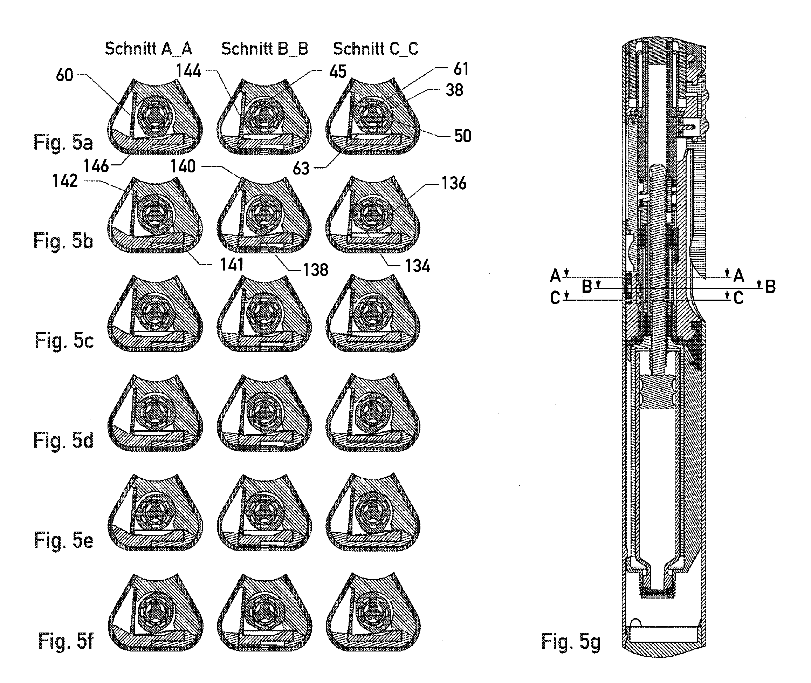

Ein zweiter Bereich der Hülse 46 weist drei axial angeordnete Nockenscheiben 61, 130 und 132 auf. Jede Nockenscheibe 61 umfasst je zwei diametral gegenüberliegende Nocken 134, 136, 138, 140, 141 und 142. Die Nockenscheiben 61 sind um je 60° zueinander verdreht. In axialer Richtung ist auf der Höhe jeder Nockenscheibe 61, 130 und 132 ein Federkontakt 63, 144 und 146 im hinteren Gehäuseteil 16 vorgesehen. Dies ist in Figur 5 dargestellt. Die Federkontakte 63, 144 und 146 sind an einer Platine 60 befestigt. Die Platine 60 ist im hinteren Gehäuseteil 16 untergebracht (s. Figur 3).

Bei einer Drehung der Hülse 46 werden die Federkontakte 63, 144 und 146 durch die Nocken 134, 136, 138, 140, 141 und 142 abwechselnd bewegt. Dadurch werden Kontakte geöffnet oder geschlossen. Figur 5 zeigt die Nocken 134, 136, 138 140, 141 und 142 und Stellungen der Federkontakte 63, 144, 146 für eine Drehbewegung gegen den Uhrzeigersinn. Für eine Drehung gegen den Uhrzeigersinn ergeben sich Stellungen, die in folgender Tabelle aufgeführt sind. Ein geschlossener Kontakt wird durch eine 1, ein geöffneter Kontakt durch eine 0 symbolisiert.

| Figur | Drehwinkel | Nockenscheibe A | Nockenscheibe B | Nockenscheibe C |
|---|---|---|---|---|
| 5 a | 0° | 1 | 0 | 0 |
| 5 b | 30° | 1 | 1 | 0 |
| 5 c | 60° | 0 | 1 | 0 |
| 5 d | 90° | 0 | 1 | 1 |
| 5 e | 120° | 0 | 0 | 1 |
| 5 f | 180° | 1 | 0 | 1 |

Ausgehend von der Stellung bei einem Drehwinkel von 0° ergibt sich bei einer Drehung im Uhrzeigersinn für die erste 30° Drehung die folgende Codierung:

| Drehwinkel | Nockenscheibe A | Nockenscheibe B | Nockenscheibe C |
|---|---|---|---|
| 0° | 1 | 0 | 0 |
| -30° | 1 | 0 | 1 |

Durch die Reihenfolge, in der sich die Kontakte öffnen (0) und schließen (1) kann die Drehrichtung der Hülse 46 eindeutig zugeordnet werden. Der Abfolge der verschiedenen Schalterstellungen ist eine korrespondierende Insulindosis zugeordnet. Der dritte Kontakt dient im Wesentlichen der Ausfallsicherheit und kann zum Erkennen von Fehlern verwendet werden.

Die Hohlwelle 50 weist dosierknopfseitig eine erste Klaue 65 auf. Diese ist in Figur 6 zu erkennen. Eine zweite Klaue 67 einer Verlängerung 62 greift in die erste Klaue 65 der Hohlwelle 50 ein. Die Verlängerung 62 ist ebenfalls hohl. Die zweite Klaue 67 befindet sich auf der der Abdeckkappe 18 zugewandten Seite der Verlängerung 62. Auf dem anderen Ende der Verlängerung 62 ist der Dosierknopf 69 befestigt. Wenn der Patient den Dosierknopf 69 dreht, folgt die Verlängerung 62 der Rotationsbewegung. Die Verlängerung 62 überträgt die Rotation über Klaue 67 auf die Klaue 65. Eine Drehung des Dosierknopfes 69 wird somit auf die Hohlwelle 50 und über die Hohlwelle auf die Mutter 40 übertragen.

Durch einen Abstand erlauben die Klauen 65 und 67 eine axiale Bewegung von Hohlwelle 50 und Verlängerung 62 zueinander. Dosierknopfseitig ist ein Absatz 71 an der zweiten Klaue 67 vorgesehen. Ab diesem Absatz 71 verjüngt sich die Verlängerung 62. Der Absatz 71 weist dosierknopfseitig eine Stirnverzahnung 70 auf.

Über die Verlängerung 62 ist eine Vorschubhülse 66 geschoben. Die Vorschubhülse 66 weist eine der Abdeckung 18 zugewandte Seite und eine dem Dosierknopf 69 zugewandte Seite auf. Abdeckkappenseitig ist an der Vorschubhülse 66 eine Stirnverzahnung 72 vorgesehen, die mit der Stirnverzahnung 70 des Absatzes 71 zusammenwirkt.

Entlang der Außenseite der Vorschubhülse 66 sind rotationssymmetrisch vier Führungsschienen 74 vorgesehen. Abdeckkappensseitig verbindet ein Ring 76 die Führungsschienen 74 mit der der Vorschubhülse 66. Der Ring 76 weist auf der dem Dosierknopf 69 zugewandten Seite eine zackenförmige Wellenbahn 78 auf. Die zackenförmige Wellenbahn 78 wird durch die Führungsschienen 74 unterbrochen. Das dem Dosierknopf 69 zugewandte Ende der Führungsschienen 74 weist eine Sägezahnform auf.

Das dem Dosierknopf 69 zugewandte Ende der Vorschubhülse 66 weist eine erste zackenförmige Struktur 80 auf, die mit einer entsprechenden zweiten zackenförmigen Struktur 82 einer Druckhülse 68 zusammenwirkt. Die Druckhülse 68 ist ebenfalls koaxial auf der Verlängerung 62 angeordnet. An dem der Abdeckkappe 18 zugewandten Ende der Druckhülse 68 sind an der Außenwand der Druckhülse 66 Vorsprünge 84 vorgesehen.

Eine Führungsbuchse 86 ist drehbar im hinteren Gehäuseteil 16 gelagert. Dazu weist die Führungsbuchse 86 auf ihrer Außenseite eine Krempe 88 auf. Korrespondierend zur Krempe 88 ist im hinteren Gehäuseteil 16 eine Ringnut 90 vorgesehen. Krempe 88 und Ringnut 90 bilden ein Gleitlager. In einer nicht gezeigten alternativen Ausgestaltung ist die Führungsbuchse 86 nicht drehbar gelagert.

Die Führungsbuchse 86 weist Führungsnuten 92 auf. Die Anzahl der Führungsnuten 92 stimmt mit der Anzahl der Vorsprünge 84 der Druckhülse 68 und der Anzahl der Führungsschienen 74 der Vorschubhülse 66 überein. Die Druckhülse 68 ist in der Führungsbuchse 86 axial bewegbar angeordnet. Ein dem Dosierknopf 69 zugewandtes Ende der Führungsbuchse weist einen Anschlag 94 für die Druckhülse 66 auf. Der Anschlag 94 ist in Figur 1 dargestellt.

Das der Abdeckkappe 18 zugewandte Ende der Führungsbuchse 86 weist eine sägezahnartige Stirnverzahnung 98 auf, die mit den sägezahnartigen Enden der Vorschubhülse 66 korrespondieren. Die Sägezähne 98 der Führungsbuchse 86 sind so ausgebildet, dass nach jeder zweiten steilen Flanke eine Führungsnut 92 angeordnet ist.

Die oben bereits erwähnte Auslösefeder 96 ist axial zwischen Hohlwelle 50 und Vorschubhülse 66 angeordnet. Die Auslösefeder 96 ist in Figur 1 dargestellt. Die Auslösefeder drückt die beiden Klauen 65 und 67 auseinander.

Die Federkonstante der Auslösefeder 96 ist größer, als die Federkonstante der Druckfeder 53. Dadurch wird in erster Näherung erst die Druckfeder 53 und dann die Auslösefeder 96 komprimiert.

Die Auslösefeder 96 und die Druckfeder 53 beaufschlagen die Vorschubhülse 66 mit einer Kraft, die in Richtung des Dosierknopfes 69 wirkt. Dadurch wird die Vorschubhülse 66 gegen die Führungsbuchse 86 gedrückt.

Wenn sich die Vorschubhülse 66 mit ihren Führungsschienen 74 in die Führungsnuten 92 der Führungsbuchse 86 eingreift, bewegt sich die Vorschubhülse 66 durch die Federkraft der Druckfeder 53 in Richtung des Abschlussteils 12. Mit der Vorschubhülse 66 bewegt sich auch die Druckhülse 68 in der Führungsbuchse 86 bis die Vorsprünge 84 der Druckhülse 68 den Anschlag 94 der Führungsnuten 92 in der Führungsbuchse 86 erreichen. Dies definiert eine erste Position der Vorschubhülse 66. Der Dosierknopf 69 ist nun zugänglich und kann gedreht und anderweitig bedient werden.

Durch Drücken des Dosierknopfes 69 wird zunächst die Druckfeder 53 komprimiert. Die Druckhülse 68 bewegt sich in Richtung der Abdeckkappe 18. Dadurch bewegen sich die Vorschubhülse 66, die Verlängerung 62 und die Hohlwelle 50 ebenfalls in diese Richtung. Wenn das leitende Rohr 56 die Metallkontakte an der Lagerbuchse 48 erreicht, ist der Schalter geschlossen.

Dadurch wird die applizierte Insulindosis gespeichert. Es wird zusätzlich ein Zeitstempel erzeugt und in einem Speicher gespeichert. Der Insulinpen befindet sich in einem Energiesparmodus.

Bei weiterem Druck auf den Dosierknopf 69 wird die Auslösefeder 96 komprimiert. Dadurch reduziert sich der Abstand zwischen den Klauen 65 und 67. Sobald die Führungsschienen 74 der Vorschubhülse 66 nicht mehr von den Führungsnuten 92 der Führungsbuchse 86 geführt werden, wird durch die Wirkung der Stirnverzahnung zwischen Druckhülse 68 und Vorschubhülse 66 die Vorschubhülse 66 um einen Sägezahn an der Führungsbuchse 86 weitergedreht.

Es gibt dort keine zu den Führungsschienen 74 der Vorschubhülse 66 korrespondierenden Führungsnuten. Wird der Dosierknopf 69 nicht mehr gedrückt entspannt sich die Auslösefeder und drückt die Vorschubhülse 66 gegen die Führungsbuchse 86. Die Vorschubhülse 66 ist in dieser Position arretiert.

In dieser Position ist der Dosierknopf 69 im Gehäuse der Anordnung versenkt. Der Dosierknopf 69 ist gegen unerwünschtes Betätigen gesichert.

Bei erneutem Drücken des Dosierknopfes 69 in Richtung der Abdeckkappe 18 wird die Auslösefeder 96 komprimiert. Die Druckhülse 68 schiebt die Vorschubhülse 66 in Richtung der Abdeckkappe 18. Wenn die Führungsschienen 74 der Vorschubhülse 66 über die steile Flanke der sägezahnförmigen Stirnverzahnung 98 der Führungsbuchse 86 geschoben werden, wird durch die Wirkung der Stirnverzahnung zwischen Druckhülse 68 und Vorschubhülse 66 die Vorschubhülse 66 um einen Sägezahn auf der Führungsbuchse 86 weitergedreht.

Nun stehen den Führungsschienen der Vorschubhülse 66 wieder korrespondierende Führungsnuten 74 der Führungsbuchse 86 gegenüber. Beim Loslassen des Dosierknopfes 69 bewegt sich durch den Federdruck die Vorschubhülse 66 in Richtung des hinteren Abschlussteils 12. Dadurch bewegt sich der Dosierknopf 69 aus dem Abschlussteil 12 heraus. Der Dosierknopf 69 ist nun wieder zugänglich und kann gedreht und anderweitig bedient werden.

An der Anordnung 10 ist ein Display 100 vorgesehen. Am Gehäuse ist weiterhin ein Clip 102 vorgesehen. Dieser dient zur Befestigung der Anordnung an einer Tasche oder dergleichen. Weiterhin ist am hinteren Gehäuseteil 16 eine Farbcodierung 108 vorgesehen. Diese ermöglicht es verschiedenfarbige Codierungen für unterschiedliche Insuline zu verwenden.

Ein Schalter 106 aktiviert die Anordnung 10 bei erstmaliger Inbetriebnahme. Es versteht sich, dass die erstmalige Aktivierung auch durch Einsetzen von Batterien vorgenommen werden kann. Die Batterien sind auswechselbar.

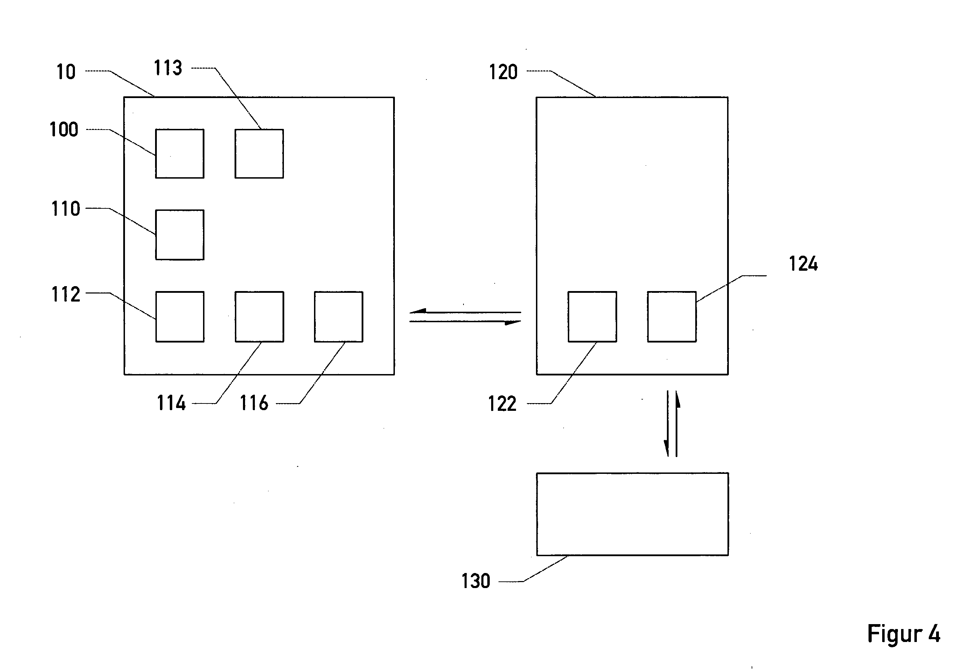

Die Applikationsanordnung 10 wird durch Betätigen des Dosierknopfes 69 ein- und ausgeschaltet. Wenn die Applikationsanordnung 10 eingeschaltet ist, werden die zuletzt applizierte Insulindosis und der Zeitpunkt der Applikation auf dem Display 100 angezeigt. Ein in Figur 4 dargestellter Zeitgeber 110 erfasst die Zeit. Ein Prozessor 112 errechnet daraus die Uhrzeit und das Datum. Zu bestimmten Zeiten wird der Inhalt des Speichers 114 über eine Funkverbindung gesendet. Das Sendesignal kann auch manuell ausgelöst werden. Hierfür ist ein Schalter 107 vorgesehen.

In Figur 4 ist schematisch ein Netzwerk dargestellt. Das Netzwerk umfasst die Applikationsanordnung 10, eine Basisstation 120 und eine Datenverarbeitungsanlage 130.

Die Applikationsanordnung umfasst den Speicher 114, eine Sende- und Empfangseinrichtung 116 und den Zeitgeber 110. Die Applikationsanordnung 10 kann mit der Basisstation 120 kommunizieren. Dazu ist in der Basisstation eine Sende- und Empfangseinrichtung 122 vorgesehen.

Die Kommunikation erfolgt bidirektional mittels Funk beispielsweise im Frequenzbereich von 868 MHz. Beim Einsetzen der Batterien sucht die Applikationsanordnung 10 nach der Basisstation 120. Dazu werden Funkwellen ausgesendet.

Die Basisstation 120 empfängt dieses Signal und sendet an die Applikationsanordnung 10 ein Signal. Daraufhin wird in der Applikationsanordnung 10 der Zeitgeber 110 in Gang gesetzt. Die Basisstation 120 übermittelt an die Applikationsanordnung 10 auch wann die nächste Kommunikation erfolgen soll. Die Applikationsanordnung 10 sendet dann zu vordefinierten Zeiten ein Signal an die Basisstation 120.

Wenn die Applikationsanordnung nicht innerhalb eines Zeitintervalls ein Signal der Basisstation 120 erhält, beendet die Applikationsanordnung 10 das Senden. Dadurch wird die Batterie der Applikationsanordnung 10 nicht unnötig belastet.

Wenn eine Kommunikation mit der Basisstation 120 zustande kommt, sendet die Applikationsanordnung 10 die gespeicherten, applizierten Insulindosen mit dem dazugehörigen Zeitstempel. Die Basisstation 120 überträgt die von der Applikationsanordnung 10 empfangenen Daten an eine Datenverarbeitungsanlage 130.

Diese Übertragung erfolgt über ein in der Basisstation 120 vorgesehenes Modem 124. Das Modem 124 arbeitet im vorliegenden Ausführungsbeispiel mit dem Mobilfunkstandard GSM. Die Basisstation 120 wählt sich über das Modem 124 in die Datenverarbeitungsanlage 130 ein. Dann erfolgt der Datenaustausch. Die Daten der Datenverarbeitungsanlage sind über ein Webinterface zugänglich. Über das Webinterface lassen sich auch Einstellungen an der Basisstation oder der der Applikationsvorrichtung vornehmen.

Eine alternative Ausgestaltung einer Applikationsanordnung ist allgemein mit 300 bezeichnet. Diese ist in Figur 7 gezeigt.

Wie im vorstehenden Ausführungsbeispiel weist die Applikationsanordnung 300 ein Gehäuse 312 mit einer Abdeckkappe 318 und einer Patrone 320 auf, welches in einen vorderen Gehäuseteil 314 und einen hinteren Gehäuseteil 316 unterteilt ist. Die Patrone 320 weist eine Membran 322 und einen Stopfen 324 auf. Die Patrone 320 sitzt auch hier in einer Adapteranordnung zur Anpassung der Patronen unterschiedlicher Hersteller und Abmessungen an die vorliegende Applikationsanordnung. Ein Schalter 490 detektiert, ob eine Adapteranordnung eingesetzt ist.

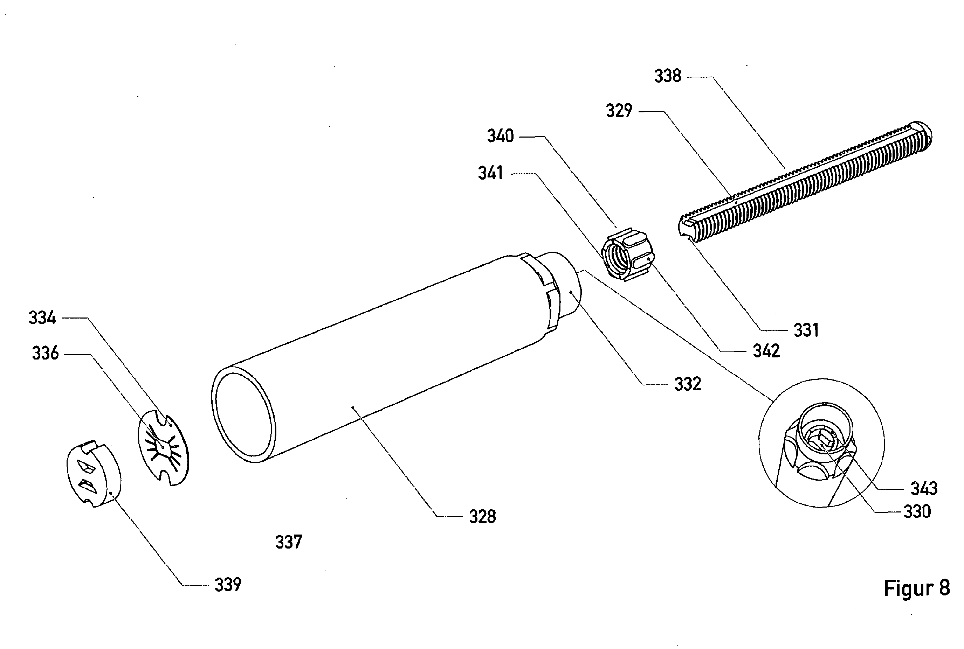

Die Adapteranordnung des vorliegenden Ausführungsbeispiels umfasst wie im vorhergehenden Ausführungsbeispiel eine Hülle 328, eine Gewindestange 338, eine Scheibe 334 und eine Scheibe 340, sowie zusätzlich einen Stempel 339. Die Komponenten der Adapteranordnung sind in Figur 8 separat noch einmal im Detail dargestellt. Die Funktionsweise der Adapteranordnung unterscheidet sich im Wesentlichen nicht von der Adapteranordnung des oben beschriebenen Ausführungsbeispiels. Der Stempel 339 ist abdeckungsseitig an der Gewindestange 338 befestigt. Dadurch wird der Druck gleichmäßig auf den Stopfen 324 verteilt.

Das dem Dosierknopf 69 zugewandte Ende der Hülle 328 ist geschlossen und weist einen mittigen Durchbruch 330 auf. Der Durchbruch 330 ist eine kreisrunde Öffnung mit zwei nach innen ragenden Radialvorsprüngen. Das Profil der Gewindestange weist zwei korrespondierende radiale Ausbuchtungen 329 und 331 auf.

Die Scheibe 334 weist eine ovale, an den Querschnitt der Gewindestange angepasste Öffnung 336 auf. Der Rand der Öffnung 336 ist teilweise in radialer Richtung geschlitzt. Dadurch entstehen kleine, federnde Blechzungen 337. Die Gewindestange 338 durch den Durchbruch 330 geführt.

Die Mutter 340 folgt der Drehung des Dosierknopfes 402 über eine nachstehend beschriebene Mechanik. Die der Abdeckung zugewandten Seite der Mutter 340 weist eine sägezahnförmige Stirnverzahnung 341 auf. Die Stirnverzahnung 341 der Mutter 340 wirkt mit einer korrespondierenden Stirnverzahnung 343 an der Hülle 328 zusammen. Die Steigung der Sägezähne entspricht der Steigung des Gewindes der Gewindestange 338. Dies gewährleistet, dass nur definierte Einheiten appliziert werden können.

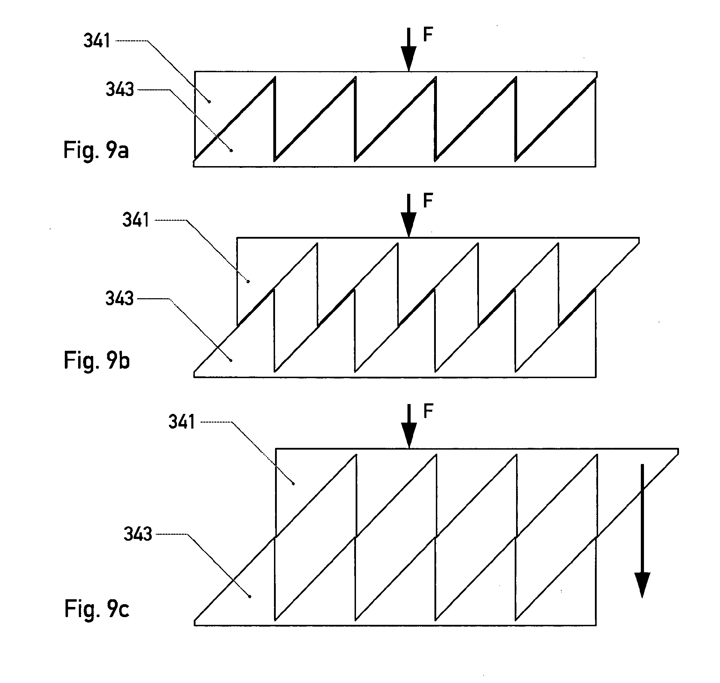

Das Funktionsprinzip der Dosierung ist in Figur 9a-c illustriert. Vor der Applikation liegen die beiden Verzahnungen 341 und 343 aufeinander. Dies ist in Figur 9 a dargestellt. Wenn die Mutter 340 auf der Gewindestange 338 gedreht wird, verschiebt sich die Verzahnung 341 gegen die Verzahnung 343. Die schrägen Flächen der Verzahnungen berühren sich jedoch weiterhin. Eine Kraft F, die in eingezeichneter Richtung auf die Gewindestange 338 einwirkt, führt zu keiner axialen Bewegung der Gewindestange. Dies ist in Figur 9b erkennbar. Wenn die Mutter 340 so weit gedreht wird, dass sich jeweils die steilen Flanken der Verzahnung gegenüberstehen, kann die Gewindestange durch die eingezeichnete Kraft F in Richtung der Abdeckung bewegt werden. Dies ist in Figur 9c dargestellt. Der Mechanismus erhöht die Applikationsgenauigkeit.

Die Abdeckkappe 318 wird von einer Röhre 350 mit im wesentlichen dreieckigen Querschnitt gebildet. Dies ist in Figur 10a gut zu erkennen. Ein Ende der dreieckigen Röhre 350 wird von einem Endstück 352 abgeschlossen. Der vordere Gehäuseteil 314 dient als Aufnahme für die Hülle 328 der Adapteranordnung 327. Der Stempel 339 und die Scheibe 334 sind in der Hülle 328 angeordnet. Die Mutter 340 und die Gewindestange 338 ragen in den hinteren Gehäuseteil 316 hinein. Dies ist in Figur 7 zu erkennen. Der Gehäuseteil 316 umfasst einen langgestreckten Korpus 356 von ebenfalls im Wesentlichen dreieckigem Querschnitt. Dies ist in Figur 10b zu erkennen. Ein Zwischenstück 354 ist zwischen dem Gehäuseteil 314 und dem Korpus 356 angeordnet.

Das Zwischenstück 354 weist einen Bajonettverschluss 358 auf, mit dem das vordere Gehäuseteil 314 befestigt ist. Das Zwischenstück 354 weist Vertiefungen 360 auf, die in Erhebungen 332 der Hülle 328 eingreifen. Die Hülle 328 ist dadurch gegen Verdrehen gesichert.

In den Korpus 356 ist ein Anzeige- und Bedienelement 372 eingepasst. Das Anzeigeund Bedienelement 372 umfasst ein Anzeigefenster 374 und ein Bedienfeld 376. Auf einer Anzeigeplatine 378 befindet sich ein Bediensensor 380, der über das Bedienfeld 376 angesprochen wird. Weiterhin befindet sich auf der Anzeigeplatine 378 ein Display 382.

Am Korpus 356 ist ein Schlitz 362 für eine Batteriehalterung 364 vorgesehen. Die Batteriehalterung 364 hält Batterien oder Akkumulatoren 366, 368 und 370, welche die Applikationsanordnung 300 mit Strom versorgen. Die Batteriehalterung 364 hält die Batterien 366, 368 und 370 in einem Batteriefach 384 in Position. Das Batteriefach 384 ist ebenfalls im Korpus 356 vorgesehen.

Ein Kontaktstreifen 386 kontaktiert den Pluspol der Batterien 366, 368 und 370 und verbindet diesen mit der Anzeigeplatine 378. Der Minuspol der Batterien 366, 368 und 370 ist über ein Federblech 388 mit der Anzeigeplatine 378 verbunden.

Ein Clip 390 ist mittels einer Schraube 392 am Batteriefach 384 befestigt. Eine Clipabdeckung 394 verdeckt die Schraube 392.

Eine Sensorpatine 396 ist mit der Anzeigeplatine 378 elektrisch verbunden. Auf der Sensorplatine 396 sind zwei Magnetsensoren 397 und 398 vorgesehen. Ein Einschaltsensor 399 ist ebenfalls auf der Platine vorgesehen. Eine Knopfeinfassung 400 schließt den Korpus 356 auf einer Seite ab.

Die eigentliche Mechanik der Applikationsanordnung umfasst von rechts nach links den Dosierknopf 402, die Knopfeinfassung 400, ein Hutelement 404, eine Krone 406, eine Gegenkrone 408, eine Ausgleichsfeder 410, einen Sicherungsring 412, einen Anschlag 414, einen Ringmagnet 416, eine Druckfeder 418, eine Welle 420, ein Kupplungselement 422 und ein Rastelement 424. Das Zusammenwirken der Komponenten ist nachfolgend anhand der Figuren 11 a und 11 b erläutert.

Das Kupplungselement 422 verbindet die Mutter 340 mit der Welle 420. Das rotationssymmetrisch um eine Längsachse der Applikationsanordnung 300 ausgebildete Kupplungselement 422 ist in dem Hohlraum, der von Anzeige- und Bedienelement 372 und Batteriefach 384 gebildet ist, drehbar gelagert.

Das Kupplungselement 422 weist eine Längsbohrung 426 auf. Die Innenwand des Kupplungselements 422 weist Längsnuten 428 auf. Die auf der Mutter 340 vorgesehenen Mitnehmer 342 sind in den Längsnuten 428 geführt. Die Mutter 340 ist entlang der Achse des Kupplungselements 422 verschiebbar und folgt einer Drehbewegung des Kupplungselements 422. Da sich die Mutter 340 auf der Gewindestange 338 befindet, bewegt sich die Mutter 340 in axialer Richtung in dem Kupplungselement 422.

Eine Welle 420 ist in dem Kupplungselement 422 geführt. Die Welle 420 weist an dem in der Kupplungselement 422 versenkten Ende radial nach außen stehende Nasen 430 auf, die mit den Nuten 428 der Kupplung 420 zusammenwirken. Die Welle 420 ist in axialer Richtung entlang der Nuten 428 verschiebbar. Die Kupplung 420 ist mit der Federkraft einer Druckfeder 418 beaufschlagt. Die Welle 420 weist dosierknopfseitig eine Verjüngung auf, an der ein Sicherungsring 412 vorgesehen ist.

Das Kupplungselement 422 weist eine ringförmig umlaufende Verdickung 431 auf. Abdeckkappenseitig weist die Verdickung 431 stirnseitig ein Sägezahnprofil 432 auf. Das Sägezahnprofil 432 wirkt mit einem entsprechenden Profil 434 des Rastelements 424 zusammen.

Durch Drehen der Welle 420, beispielsweise im Uhrzeigersinn, verschieben sich die Sägezähne gegeneinander und das Kupplungselement 422 bewegt sich gegen die Federkraft der Druckfeder 418. Sobald sich die steilen Flanken der Sägezähne gegenüberstehen, entspannt sich die Druckfeder 418 und bewegt das Kupplungselement 422 gegen das Rastelement 424.

Diese Bewegung ist sowohl hörbar als auch fühlbar. Das Rastelement 424 und das Kupplungselement 422 bilden ein akustisches und haptisches Feedback für das Einstellen einer zu applizierenden Dosis durch Drehung.

Für ein Feedback bei Drehung in der anderen Richtung, beispielsweise gegen den Uhrzeigersinn, weist das Rastelement 424 auf der gegenüberliegenden Seite eine weitere Stirnverzahnung 436 auf. Diese ist gegenläufig ausgebildet und wirkt mit einem Sägezahnprofil 438 zusammen, welches dosierknopfseitig an dem Zwischenstück 354 vorgesehen ist. Bei Drehung im Uhrzeigersinn verhaken sich die Zähne der Stirnverzahnung 436 und des Sägezahnprofils 438. Die Kupplungselement 422 und das Rastelement drehen sich gegeneinander und liefern das Feedback. Bei einer Drehung gegen den Uhrzeigersinn, dreht sich der Effekt gerade um. Rastelement 424 und Kupplungselement 422 verkeilen sich und das Rastelement bewegt sich gegen das Zwischenstück 354, wobei die entsprechenden Verzahnungen ein akustisches und haptisches Feedback geben.

An dem Kupplungselement 422 sind radial umlaufend sechs Magnete 440, 442, 444, 446, 448 und 450 angeordnet. Dabei handelt es sich um Stabmagnete. Die Ausrichtung ist derart gewählt, dass umlaufend immer ein Nord- und ein Südpol alternierend angeordnet sind. Zwischen je zwei benachbarten Polen bildet sich ein Magnetfeld aus, welches von Nordpol zu Südpol verläuft.

Die Orientierung der Magnetfelder wird von den zwei Magnetsensoren 397 und 398 auf der Sensorplatine 396 detektiert und ausgewertet. Das Prinzip ist in Figuren 12a-d gezeigt. Figur 12a zeigt einen Schnitt in der Ebene der Magnete. Auf der Sensorplatine 396 sind die Magnetsensoren 397 und 398 angeordnet. Die Magnetsensoren 397 und 398 reagieren darauf, in welcher Richtung ein Magnetfeld die Sensorfläche durchdringt. Die Signale, welche von den Magnetsensoren erzeugt werden, werden anhand einer Drehung im Uhrzeigersinn erläutert.

In Figur 12b wird der Magnetsensor 397 von einem Magnetfeld zwischen den Magneten 440 und 442 durchdrungen. Das Magnetfeld ist im Schaubild von oben nach unten ausgerichtet. Der Magnetsensor 397 detektiert ein Signal, welches einer Null zugeordnet wird. Der Magnetsensor 398 wird von einem Magnetfeld zwischen den Magneten 450 und 440 durchdrungen. Hier ist das Magnetfeld in der Zeichnung von unten nach oben ausgerichtet. Der Magnetsensor 398 detektiert ein Signal, welches einer 1 zugeordnet wird.

In Figur 12c sind die Magnetfelder undefiniert. Die Sensoren behalten ihr vorheriges Signal bei. Bei einer weiteren Drehung im Uhrzeigersinn dreht sich die Magnetfeldrichtung in den beiden Magnetsensoren 397 und 398 um. Dies ist in Figur 12d dargestellt.

Die Ausgangssignale der Magnetsensoren 397 und 398 ändern sich. Bei jeder Drehung um 60° ändert sich die Orientierung der Magnetfelder durch die Sensoren und damit deren Ausgangssignal. Die Abfolge der Änderungen wird von einer Elektronik ausgewertet und in eingestellte Einheiten umgerechnet, welche dann auf der Anzeige 382 dargestellt werden. Zur Veranschaulichung ist eine Tabelle eingefügt.

| Im Uhrzeigersinn | Magnetsensor | Magnetfeldrichtung | Ausgangssignal |
|---|---|---|---|
| Figur 12 b | Magnetsensor 397 | runter | 0 |
| | Magnetsensor 398 | hoch | 1 |
| Figur 12 c | Magnetsensor 397 | | 0 |
| | Magnetsensor 398 | | 1 |
| Figur 12 d | Magnetsensor 397 | hoch | 0 |
| | Magnetsensor 398 | runter | 1 |
| Figur 12 a | Magnetsensor 397 | | 0 |
| | Magnetsensor 398 | | 1 |

Entsprechend funktioniert das Zählen in der anderen Drehrichtung. Es ist denkbar, Hallsensoren zu verwenden. Anders als bei mechanischen Teilen zum Ermitteln der eingestellten Dosis verschleißen die Komponenten bei diesem Ausführungsbeispiel praktisch nicht. Dadurch wird die Genauigkeit und die Lebensdauer der Applikationsanordnung 300 weiter verbessert.

Die Druckfeder 418 ist zwischen dem Kupplungselement 422 und dem Ringmagneten 416 eingespannt. Der Anschlag 414 weist abdeckungsseitig ein verbreitertes Ende 452 mit dem Ringmagneten 416 und dosierknopfseitig ein schmaleres Ende auf. Zwischen dem schmaleren Ende 454 und dem verbreiterten Ende 452 ist ein Absatz 456 gebildet. Das schmalere Ende 454 ist bis zum Absatz 456 in der Gegenkrone 408 versenkt.

Die Gegenkrone 408 ist mit einem dosierknopfseitigen Ende am Dosierknopf 402 befestigt. In der Gegenkrone 408 sind Führungsnuten 460 vorgesehen. Diese brechen nicht bis zu dem der Abdeckung zugewandten Ende 462 durch. In diesen Führungsnuten laufen korrespondierende Führungen 464 der Welle 420. Die Welle 420 wird von einer Ausgleichsfeder 410 zwischen Welle 420 und Dosierknopf 402 beaufschlagt. Der Sicherungsring 412 begrenzt den Hub der Führungen 464 in den Führungsnuten 464. Die Ausgleichsfeder 410 sorgt dafür, dass die Mutter 420 verlässlich gegen die Hülle 328 drückt.

Der Dosierknopf 402 ist in der Knopfeinfassung 400 versenkbar. In versenkter Position ist die Druckfeder 418 gespannt. In ausgefahrener Position entspannt sich die Druckfeder 418. Dadurch wird die Welle 420 etwas aus der Kupplungselement 422 gedrückt. Zwischen der Mutter 340 und der Welle 420 entsteht dann ein Spalt.

Durch Drehen des Dosierknopfes 402 dreht sich die daran befestigte Gegenkrone 408. Über die Führungsnuten 460 und die Führungen 464 wird die Drehung auf die Welle 420 übertragen. Durch eine Drehbewegung des Dosierknopfes 402 dreht sich auch die Welle 420 im Uhrzeigersinn. Dadurch verschiebt sich die Mutter 340 in dem Kupplungselement 422 in Richtung der Welle 420. Der Spalt zwischen Welle 420 und Mutter 340 wird reduziert. Der Spalt zwischen der Hülle 328 und der Mutter 340 vergrößert sich.

Durch Drücken des Dosierknopfes in Richtung der Abdeckkappe verschiebt sich die Welle 420 und die Druckfeder 418 wird zusammengedrückt. Der Spalt zwischen Welle 420 und Mutter 340 verringert sich. Sobald die Welle 420 die Mutter 340 berührt, bewegt sich diese bei fortgesetztem Druck auf die Welle 420 in Richtung der Abdeckkappe. Da die Mutter 340 über das Gewinde mit der Gewindestange 338 verbunden ist, wird diese ebenfalls verschoben. Die Bewegung endet, wenn der Spalt zwischen Mutter 340 und Hülle 328 verschwindet. Danach wird nur noch die Druckfeder 418 komprimiert.

Dadurch bewegt sich der Ringmagnet 416 bis er den Einschaltsensor 399 auslöst. Im vorliegenden Ausführungsbeispiel umfasst der Einschaltsensor einen Reedkontakt. Der Einschaltsensor gibt einerseits ein Signal, das Insulin appliziert wurde. Andererseits dient er auch zum Ein- und Ausschalten der Applikationsanordnung 300.

Die Krone 408 weist an ihrem dem Dosierknopf 402 zugewandten Ende eine zackenförmige Struktur 466 auf. Entlang der Außenseite der Krone 408 sind rotationssymmetrisch vier Führungsschienen 468 vorgesehen. Eine ringförmige Erhebung 470 verbindet die Führungsschienen 468 der Krone 408. Die ringförmige Erhebung 470 weist auf der dem Dosierknopf 402 zugewandten Seite eine zackenförmige Wellenbahn auf. Die zackenförmige Wellenbahn wird durch die Führungsschienen 468 unterbrochen. Das dem Dosierknopf 402 zugewandte Ende der Führungsschienen 468 ist der zackenförmige Struktur 466 angepasst.

Im Dosierknopf 402 ist eine Druckhülse 474 befestigt, welche an ihrem der Abdeckkappe zugewandten Ende eine zackenförmige Struktur 476 aufweist. Die zackenförmige Struktur 476 und die zackenförmige Struktur 466 bilden eine Stirnverzahnung. An der Druckhülse 474 sind an der Außenwand Vorsprünge 478 vorgesehen. Diese korrespondieren mit den Führungsschienen 468 der Krone 408.

Das Hutelement 404 ist drehbar im hinteren Gehäuseteil 316 gelagert. Dazu weist das Hutelement 404 auf seiner Außenseite eine umlaufende Krempe auf. Korrespondierend zur Krempe ist an der Knopfeinfassung 400 eine Ringnut 482 vorgesehen. Krempe und Ringnut 482 bilden ein Gleitlager.

Das Hutelement 404 weist Führungsnuten 488 auf. Die Anzahl der Führungsnuten 488 entspricht der Anzahl der Vorsprünge 478 der Druckhülse 474 und der Anzahl der Führungsschienen 468 der Krone 408. Die Druckhülse 474 ist im Hutelement 404 axial beweglich angeordnet.

Das der Abdeckkappe zugewandte Ende des Hutelementes 404 mit der Krempe weist am inneren Rand eine sägezahnartige Stirnverzahnung 486 auf, die mit den Enden der Führungsschienen 468 zusammenwirkt. Die Sägezähne der Stirnverzahnung 486 des Hutelements 402 sind so ausgebildet, dass nach jeder zweiten steilen Flanke eine Führungsnut 488 angeordnet ist.

Die Federkonstante der Ausgleichsfeder 410 ist größer, als die Federkonstante der Druckfeder 418.

Im ausgeschalteten Zustand der Applikationsanordnung 300 ist der Dosierknopf 402 in der Knopfeinfassung 400 versenkt. Der Dosierknopf 402 ist gegen unerwünschtes Betätigen gesichert. Eine auf der Innenseite der Krone 406 umlaufende Kante und eine am Ende 462 der Gegenkrone vorgesehene Verdickung verhindern das Herausrutschen des Dosierknopfes 402.

Zum Entriegeln wird der Dosierknopf 402 in Richtung der Abdeckkappe gedrückt. Dabei schiebt die Druckhülse 474 die Krone 406 soweit in Richtung der Abdeckung, dass die Führungsschienen 468 der Krone 406 über die steile Flanke der sägezahnförmigen Stirnverzahnung 486 des Hutelementes 404 geschoben werden. Durch die Wirkung der Stirnverzahnung zwischen Druckhülse 474 und Krone 406, wird die Krone 406 um einen Sägezahn auf dem Hutelement 404 weitergedreht. Nun stehen den Führungsschienen 468 der Krone 406 korrespondierende Führungsnuten des Hutelementes 404 gegenüber.

Durch den Federdruck der Druckfeder 418 bewegt sich die Krone 406 beim Loslassen des Dosierknopfes 402 in Richtung der Knopfeinfassung 400. Dadurch bewegt sich der Dosierknopf 402 aus der Knopfeinfassung 400 heraus. Der Dosierknopf 402 ist nun zugänglich und kann gedreht und anderweitig bedient werden.

Nach Einstellen der Dosis wird durch Drücken des Dosierknopfes 402 die Krone 406 in Richtung der Abdeckkappe geschoben. Dadurch wird auch die Welle 420 bewegt. Die Welle 420 drückt auf die Mutter 420, die ihre Bewegung auf die Gewindestange 388 überträgt. Die Gewindestange 388 drückt zum Applizieren von Insulin auf den Stempel der Patrone. Der Ringmagnet 416 bewegt sich in Richtung der Abdeckkappe, wodurch der Ein- und Ausschaltsensor 399 ausgelöst wird. Dadurch wird die applizierte Insulindosis gespeichert. Es wird ein Zeitstempel erzeugt. Dieser Zeitstempel wird in einem Speicher abgelegt. Der Insulinpen wird in einen Energiesparmodus versetzt, bei dem das Display ausgeschaltet ist.

Sobald die Mutter 420 an die Hülle 328 anschlägt, wird durch Drücken des Dosierknopfes 420 die Druckfeder 418 komprimiert und die Krone 406 bewegt sich weiter in Richtung der Abdeckkappe. Sobald die Führungsschienen 468 der Krone 406 nicht mehr von den Führungsnuten 488 des Hutelementes 404 geführt werden, wird durch die Wirkung der Stirnverzahnung zwischen Druckhülse 474 und Krone 406 die Krone 406 um einen Sägezahn am Hutelement 404 weitergedreht. Es gibt dort keine zu den Führungsschienen 468 der Krone 406 korrespondierenden Führungsnuten.

Wird der Dosierknopf 402 nicht mehr gedrückt, entspannt sich die Druckfeder 418 und drückt die Krone 406 gegen das Hutelement 404. Die Krone 406 ist arretiert. In dieser Position ist der Dosierknopf 402 im Gehäuse der Applikationsanordnung 300 versenkt. Der Dosierknopf 402 ist gegen unerwünschtes Betätigen gesichert.

Die Applikationsanordnung 300 verfügt über die gleichen elektronischen Möglichkeiten, wie die Anordnung 10.

## Patentansprüche

1. Anordnung (10, 300) zum Applizieren einer ausgewählten Dosis Insulin oder anderer Medikamente aus einer Patrone (20, 320) durch Vorschub eines in der Patrone verschiebbar geführten Stopfens (24, 324), enthaltend
(a) ein in Vorschubrichtung bewegliches, erstes Gewindelement zum Bewegen des Stopfens, und
(b) ein zum Einstellen der ausgewählten Dosis drehbar mit dem ersten Gewindeelement verschraubtes, zweites Gewindeelement mit welchem der Hub des ersten Gewindeelementes begrenzbar ist,
**dadurch gekennzeichnet dass**
(a) das erste und das zweite Gewindeelement in einer Adapteranordnung zur Anpassung von Patronen unterschiedlicher Abmessungen und Inhalte enthalten ist, wobei
(b) die Gewindesteigung des ersten und zweiten Gewindeelements an die Abmessungen und/oder den Inhalt der Patrone angepasst ist.

2. Anordnung nach Anspruch 1, **gekennzeichnet durch** einen drehbar gelagerten Dosierknopf (69, 402), und Mittel zum Übertragen einer Rotationsbewegung des Dosierknopfes (69, 402) auf das zweite Gewindeelement.

3. Anordnung nach Anspruch 2, **dadurch gekennzeichnet, dass** der Dosierknopf (69, 402) arretierbar ist.

4. Anordnung nach Anspruch 2 oder 3, **dadurch gekennzeichnet, dass** ein Gehäuse vorgesehen ist, in welchem der Dosierknopf (69, 402) gegen die Federkraft einer Druckfeder in dem Gehäuse versenk- und dort lösbar einrastbar ist.

5. Anordnung nach einem der vorgehenden Ansprüche, **dadurch gekennzeichnet, dass**
(a) das erste Gewindeelement von einer Gewindestange (38, 338) mit Außengewinde gebildet ist,
(b) die Adapteranordnung eine Hülle (28, 328) umfasst, die einseitig zur Aufnahme einer Patrone (20, 320) offen ist und am anderen Ende einen Durchbruch (30, 330) aufweist, durch den die Gewindestange (38, 338) verdrehsicher geführt ist, und
(c) das zweite Gewindeelement von einer außerhalb der Hülle (28, 328) auf die Gewindestange (38, 338) aufgeschraubten Mutter (40, 340) gebildet ist, deren axiale Lage durch Verdrehen einstellbar ist.

6. Anordnung nach Anspruch 5, **gekennzeichnet durch** Sicherungsmittel (34, 334) zum Blockieren einer Bewegung der Gewindestange (38, 338) entgegen der Vorschubrichtung.

7. Anordnung nach Anspruch 6, **dadurch gekennzeichnet, dass** die Sicherungsmittel (34, 334) von einer Scheibe (34, 334) gebildet sind, die mit einer Öffnung zwischen der Stirnseite der Patrone und der inneren Stirnseite der Hülle (28) auf die Gewindestange (38) aufgesteckt ist und welche im Bereich der Öffnung flexible Zungen aufweist, welche in das Gewinde der Gewindestange (38) eingreifen.

8. Anordnung nach einem der vorgehenden Ansprüche, **gekennzeichnet durch** eine Sensoranordnung zum kontaktlosen Erfassen der eingestellten Dosis.

9. Anordnung nach Anspruch 8, **dadurch gekennzeichnet, dass** die Sensoranordnung enthält:
(a) Magnete (440, 442, 444, 446, 448, 450) an einer der zum Einstellen der Dosis rotierenden Komponenten,
(b) Magnetsensoren (397, 398) zum Detektieren einer Rotationsbewegung der Magnete und
(c) Auswerte- und/oder Anzeigemittel zum Auswerten und/oder Anzeigen einer der detektierten Bewegung entsprechenden Dosis, die aus der detektierten Rotationsbewegung ermittelt wurde.

10. Adapteranordnung für die Anordnung gemäß Anspruch 1 zur Aufnahme von Patronen unterschiedlicher Abmessungen und Inhalte für Applikationsvorrichtungen für Insulin oder andere Medikamente durch Vorschub eines in der Patrone verschiebbar geführten Stopfens (24, 324), **gekennzeichnet durch**
(a) ein in Vorschubrichtung bewegliches, erstes Gewindelement zum Bewegen des Stopfens und
(b) ein zum Einstellen der ausgewählten Dosis drehbar mit dem ersten Gewindeelement verschraubtes, zweites Gewindeelement, mit welchem der Hub des ersten Gewindeelements begrenzbar ist, wobei
(c) die Gewindesteigung des ersten und zweiten Gewindeelements an die Abmessungen und/oder den Inhalt der Patrone angepasst ist.

11. Adapteranordnung nach Anspruch 10, **dadurch gekennzeichnet, dass**
(a) das erste Gewindeelement von einer Gewindestange (38, 338) mit Außengewinde gebildet ist,
(b) eine Hülle (28, 328) vorgesehen ist, die einseitig zur Aufnahme einer Patrone (20, 320) offen ist und am anderen Ende einen Durchbruch (30, 330) aufweist, durch den die Gewindestange (38, 338) verdrehsicher geführt ist, und
(c) das zweite Gewindeelement von einer außerhalb der Hülle (28, 328) auf die Gewindestange (38, 338) aufgeschraubten Mutter (40, 340) gebildet ist, deren axiale Lage durch Verdrehen einstellbar ist.

## Claims

1. An arrangement (10, 300) for administering a selected dosage of insulin or other drugs from a cartridge (20, 320) by advancing a plug (24, 324) guided in said cartridge in a manner so as to be able to be slided, containing
(a) a first threaded element for moving the plug, said element being able to be moved in the advance direction, and
(b) a second threaded element for adjusting the selected dosage, rotatably screwed to the first threaded element and able to limit the lift of said first threaded element,
**characterised in that**
(a) the first and second threaded elements are contained in an adaptor arrangement for adapting cartridges of different dimensions and contents, wherein
(b) the thread pitch of the first and second threaded elements is adapted to the dimensions and/or the content of the cartridge.

2. The arrangement according to claim 1, **characterised by** a rotatably supported dosing button (69, 402) and means for transferring a rotational movement of the dosing button (69, 402) to the second threaded element.

3. The arrangement according to claim **2, characterised in that** the dosing button (69, 402) can be locked.

4. The arrangement according to claim 2 or 3, **characterised in that** a housing is provided into which the dosing button (69, 402) can be pressed against the force of a pressure spring inside the housing and be releasably locked into place there.

5. The arrangement according to any one of the preceding claims, **characterised in that**
(a) the first threaded element is formed by a threaded rod (38, 338) having an external thread,
(b) the adaptor arrangement comprises an enclosure (28, 328) being open on one side for receiving a cartridge (20, 320) and having an aperture (30, 330) at its other end for guiding the threaded rod (38, 338) therethrough in a manner so as to be safe from unintended rotation, and
(c) the second threaded element is formed by a nut (40, 340) screwed onto the threaded rod outside the enclosure (28, 328), the axial position of said nut being able to be adjusted by rotation.

6. The arrangement according to claim 5, **characterised by** a securing means (34, 334) for blocking a movement of the threaded rod (38, 338) opposite the advance direction.

7. The arrangement according to claim 6, **characterised in that** the securing means (34, 334) are formed by a disc (34, 334) attached to the threaded rod (38) with an opening between the front side of the cartridge and the inner front side of the enclosure (28) and having flexible tongues in the region of the opening, said tongues engaging with the thread of the threaded rod (38).

8. The arrangement according to any one of the preceding claims, **characterised by** a sensor arrangement for contactless sensing of the adjusted dosage.

9. The arrangement according to claim 8, **characterised in that** the sensor arrangement contains:
(a) magnets (440, 442, 444, 446, 448, 450) on one of the components rotating for adjusting the dosage,
(b) magnetic sensors (397, 398) for detecting a rotational movement of the magnets and
(c) evaluation and/or display means for evaluating and/or displaying a dosage corresponding to the detected movement, which has been determined from the detected rotational movement.

10. An adaptor arrangement for the arrangement according to claim 1 for receiving cartridges of different dimensions and contents for administering devices for insulin or other drugs by advancing a plug (24, 324) guided in said cartridge in a manner so as to be able to be slided,
**characterised by**
(a) a first threaded element for moving the plug, said element being able to be moved in the advance direction, and
(b) a second threaded element for adjusting the selected dosage, rotatably screwed to the first threaded element and able to limit the lift of the first threaded element, wherein
(c) the thread pitch of the first and second threaded elements is adapted to the dimensions and/or the content of the cartridge.

11. The adaptor arrangement according to claim 10, **characterised in that**
(a) the first threaded element is formed by a threaded rod (38, 338) having an external thread,
(b) an enclosure (28, 328) is provided which is open on one side for receiving a cartridge (20, 320) and which has an aperture (30, 330) at its other end for guiding the threaded rod (38, 338) therethrough in a manner so as to be safe from unintended rotation, and
(c) the second threaded element is formed by a nut (40, 340) screwed onto the threaded rod outside the enclosure (28, 328), the axial position of said nut being able to be adjusted by rotation.

## Revendications

1. Dispositif (10, 300) destiné à l'administration d'une dose d'insuline sélectionnée ou d'autres médicaments à partir d'une cartouche (20, 320) par l'avancement d'un bouchon (24, 324) guidé de façon coulissante dans la cartouche, contenant
(a) un premier élément fileté, mobile dans la direction d'avancement, destiné à déplacer le bouchon, et
(b) un deuxième élément fileté, vissé de façon rotative avec le premier élément fileté pour le réglage de la dose sélectionnée, avec lequel la course du premier élément fileté peut être limitée,
**caractérisé en ce que**
(a) le premier et le deuxième élément fileté sont contenus dans un dispositif d'adaptation destiné à l'adaptation de cartouches de dimensions et de contenus différents,
(b) le pas de filetage du premier et du deuxième élément fileté étant adapté aux dimensions et/ou au contenu de la cartouche.

2. Dispositif selon la revendication 1, **caractérisé par** une tête de dosage (69, 402) supportée en rotation, et par des moyens destinés à la transmission d'un mouvement de rotation de la tête de dosage (69, 402) au deuxième élément fileté.

3. Dispositif selon la revendication 2, **caractérisé en ce que** la tête de dosage (69, 402) peut être arrêtée.

4. Procédé selon la revendication 2 ou 3, **caractérisé en ce qu**'il est prévu un boîtier dans lequel la tête de dosage (69, 402) peut être encastrée, contre la force de ressort d'un ressort de pression dans le boîtier, et y être encliquetée de façon amovible.

5. Procédé selon une des revendications précédentes, **caractérisé en ce que**
(a) le premier élément fileté est formé d'une tige filetée (38, 338) avec un filet extérieur,
(b) le dispositif d'adaptation comprend une enveloppe (28, 328) qui est ouverte d'un côté pour loger une cartouche (20, 320) et qui présente à l'autre extrémité une traversée (30, 330) à travers laquelle la tige filetée (38, 338) est guidée bloquée en rotation, et
(c) le deuxième élément fileté est formé d'un écrou (40, 340) vissé à l'extérieur de l'enveloppe (28, 328) sur la tige filetée (38, 338) et dont la position axiale peut être réglée par rotation.

6. Procédé selon la revendication 5, **caractérisé par** des moyens de blocage (34, 334) destinés à bloquer un mouvement de la tige filetée (38, 338) opposé à la direction d'avancement.

7. Procédé selon la revendication 6, **caractérisé en ce que** les moyens de blocage (34, 334) sont formés d'un disque (34, 334) qui est enfiché sur la tige filetée (38) avec une ouverture entre le côté frontal de la cartouche et le côté frontal intérieur de l'enveloppe (28), et qui présente, dans la zone de l'ouverture, des languettes flexibles qui engrènent dans le filet de la tige filetée (38).

8. Procédé selon une des revendications précédentes, **caractérisé par** un dispositif de capteur destiné à la détection sans contact de la dose réglée.

9. Procédé selon la revendication 8, **caractérisé en ce que** le dispositif de capteur contient :
(a) des aimants (440, 442, 444, 446, 448, 450) sur un des composants rotatifs destinés au réglage de la dose,
(b) des capteurs magnétiques (397, 398) destinés à la détection d'un mouvement de rotation des aimants et
(c) des moyens d'analyse et/ou d'affichage pour l'analyse et/ou l'affichage d'une dose correspondant au mouvement détecté qui a été déterminée à partir du mouvement de rotation détecté.

10. Dispositif d'adaptation pour le dispositif selon la revendication 1, destiné à loger des dimensions et des contenus différents pour des équipements d'administration pour de l'insuline ou d'autres médicaments par l'avancement d'un bouchon (24, 324) guidé de façon coulissante dans la cartouche, **caractérisé par**
(a) un premier élément fileté, mobile dans la direction d'avancement, destiné au déplacement du bouchon et
(b) un deuxième élément fileté, vissé de façon rotative avec le premier élément fileté pour le réglage de la dose sélectionnée, avec lequel la course du premier élément fileté peut être limitée,
(c) le pas de filetage du premier et du deuxième élément fileté étant adapté aux dimensions et/ou au contenu de la cartouche.

11. Dispositif d'adaptation selon la revendication 10, **caractérisé en ce que**
(a) le premier élément fileté est formé d'une tige filetée (38, 338) avec un filet extérieur,
(b) il est prévu une enveloppe (28, 328) qui est ouverte d'un côté pour loger une cartouche (20, 320) et qui présente à l'autre extrémité une traversée (30, 330) à travers laquelle la tige filetée (38, 338) est guidée bloquée en rotation, et
(c) le deuxième élément fileté est formé d'un écrou (40, 340) vissé à l'extérieur de l'enveloppe (28, 328) sur la tige filetée (38, 338) et dont la position axiale peut être réglée par rotation.
